(19) Europäisches Patentamt / European Patent Office / Office européen des brevets

(11) **EP 2 490 653 B1**

(12) **EUROPÄISCHE PATENTSCHRIFT**


(45) Veröffentlichungstag und Bekanntmachung des Hinweises auf die Patenterteilung:
**11.01.2017 Patentblatt 2017/02**

(21) Anmeldenummer: **10766067.2**

(22) Anmeldetag: **21.10.2010**

(51) Int Cl.:
***A61K 8/73*** $^{(2006.01)}$ ***A61Q 5/06*** $^{(2006.01)}$
***A61K 8/81*** $^{(2006.01)}$

(86) Internationale Anmeldenummer:
**PCT/EP2010/065851**

(87) Internationale Veröffentlichungsnummer:
**WO 2011/048172 (28.04.2011 Gazette 2011/17)**


(54) **MITTEL ZUR TEMPORÄREN VERFORMUNG KERATINHALTIGER FASERN, ENTHALTEND EINE NICHTIONISCHE, MITTELS PROPYLENOXID MODIFIZIERTE STÄRKE UND EIN FILMBILDENDES UND/ODER FESTIGENDES KATIONISCHES POLYMER**

COMPOSITION FOR THE TEMPORARY SHAPING OF KERATINIC FIBRES COMPRISING A NONIONIC PROPYLENEOXIDE-MODIFIED STARCH AND A FILM-FORMING OR FIXING CATIONIC POLYMER

PRODUIT POUR FIBRES KÉRATINIQUES, CONTENANT AU MOINS UN AMIDON NON IONIQUE, MODIFIÉ PAR DE L’OXYDE DE PROPYLÈNE, ET AU MOINS UN POLYMÈRE SUPPLÉMENTAIRE CATIONIQUE FILMOGÈNE ET/OU FIXATEUR


(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.10.2009 DE 102009045925**
**22.10.2009 DE 102009045933**

(43) Veröffentlichungstag der Anmeldung:
**29.08.2012 Patentblatt 2012/35**



(73) Patentinhaber: **Henkel AG & Co. KGaA**
**40589 Düsseldorf (DE)**

(72) Erfinder:
- **MÜLLER, Burkhard**
  **21075 Hamburg (DE)**
- **KAFTAN, Pamela**
  **22525 Hamburg (DE)**
- **BAYERSDÖRFER, Rolf**
  **22589 Hamburg (DE)**
- **REINEKING, Miriam**
  **22769 Hamburg (DE)**
- **SCHWEINSBERG, Matthias**
  **22769 Hamburg (DE)**
- **RÖNISCH, Ralf**
  **42349 Wuppertal (DE)**
- **SCHRIEFERS, Mathias**
  **41239 Mönchengladbach (DE)**
- **DOGAN, Carine**
  **F-91270 Vigneux sur Seine (FR)**
- **KNAPPE, Thorsten**
  **22869 Schenefeld (DE)**



(56) Entgegenhaltungen:
**EP-A1- 0 659 394** **EP-A2- 0 948 958**
**EP-A2- 0 948 959** **EP-A2- 0 948 960**
**EP-A2- 1 317 916**

- **T.E. Gottschalck, J.E. Bailey: "International Cosmetic Ingredient Dictionary and Handbook", 2008, The Cosmetic, Toiletry and Fragrance Association, Washington, D.C., XP002627782, ISBN: 1-882621-43-3 Bd. 3, Seiten 3187-3215, Seite 3187 - Seite 3192 Seite 3214 - Seite 3215**


Bemerkungen:
Die Akte enthält technische Angaben, die nach dem Eingang der Anmeldung eingereicht wurden und die nicht in dieser Patentschrift enthalten sind.


Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



EP 2 490 653 B1

**Beschreibung**

Die vorliegende Erfindung betrifft Mittel zur Haarbehandlung, enthaltend eine Kombination aus mindestens einer nichtionischen, mittels Propylenoxid modifizierten Stärke mit mindestens einem filmbildenden kationischen und/oder festigenden Polymer, die Verwendung dieser Mittel zur temporären Verformung und/oder zur Pflege keratinhaltiger Fasern und Aerosolhaarsprays/- schäume auf Basis dieser Mittel.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, die einer permanenten oder temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Haarschäume, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Insbesondere Haargele und Haarwachse werden allerdings in der Regel nicht direkt auf das Haar appliziert, sondern mittels eines Kamms oder der Hände im Haar verteilt.

Die wichtigste Eigenschaft eines Mittels zur temporären Verformung keratinischer Fasern, im Folgenden auch Stylingmittel genannt, besteht darin, den behandelten Fasern in der erzeugten Form einen möglichst starken Halt zu geben. Handelt es sich bei den keratinischen Fasern um menschliche Haare, spricht man auch von starkem Frisurenhalt oder vom hohen Haltegrad des Stylingmittels. Der Frisurenhalt wird im Wesentlichen durch die Art und Menge des eingesetzten synthetischen Polymers bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar sein.

Um den unterschiedlichen Anforderungen gerecht zu werden, wurde bereits eine Vielzahl von synthetischen Polymeren entwickelt, die in Stylingmitteln zur Anwendung kommen. Die Polymere lassen sich in kationische, anionische, nichtionische und amphotere filmbildende und/oder festigende Polymere unterteilen. Idealerweise ergeben die Polymere bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen.

Stylingmittel zu entwickeln, die alle gewünschten Eigenschaften in Kombination aufweisen, bereitet nach wie vor Schwierigkeiten. Insbesondere gilt dies für die Kombination von starkem Halt einerseits und einfacher, gleichmäßiger Applikation auf die keratinhaltigen Fasern andererseits.

Aufgabe der vorliegenden Erfindung war es daher, ein Mittel zur temporären Verformung und/oder zur Pflege keratinischer Fasern zur Verfügung zu stellen, das sich durch einen hohen Haltegrad bzw. durch eine hohe Pflegewirkung auszeichnet und insbesondere eine hervorragende Handhabbarkeit während der Applikation auf die keratinhaltigen Fasern besitzt.

Es wurde nunmehr überraschenderweise gefunden, dass dies durch eine Kombination spezieller Polymere erreicht werden kann. Ferner gelang es im Rahmen spezieller Ausführungsformen der Erfindung zusätzlich zu diesen hervorragendenden Eigenschaften Zusammensetzungen ohne Trübung bereitzustellen. Die Trübungsfreiheit ist insbesondere im Rahmen der Bereitstellung von Aerosolzusammensetzungen von Belang, da feste Schwebeteilchen zu einer Verstopfung der Austrittsdüse der Aerosolverpackung führen können. Generell besteht bei trüben und dünnflüssigen Zusammensetzungen zusätzlich die Gefahr der Sedimentation, die sich nachteilig auf die Lagerstabilität der Zusammensetzung auswirkt.

Ein erster Gegenstand der vorliegenden Erfindung ist daher ein Mittel zur Behandlung keratinhaltiger Fasern, insbesondere menschlichem Haar, entsprechend Anspruch 1.

Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten

kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser oder Wasser/Alkohol-Gemischen besitzen, um in dem erfindungsgemäßen Mittel in vollständig gelöster Form vorzuliegen. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden.

Festigende Polymere tragen zum Halt und/oder zum Aufbau des Haarvolumens und der Haarfülle der Gesamtfrisur bei. Diese Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Als eine Testmethode für die festigende Wirkung eines Polymers werden häufig der so genannte curl-retention - Test oder der Dreipunkt-Biegetest angewendet.

Gemäß allen folgenden Formeln steht eine chemische Bindung, die mit dem Symbol * gekennzeichnet ist, für eine freie Valenz des entsprechenden Strukturfragments. Zur Kompensation der positiven Polymerladung in dem erfindungsgemäßen Mittel dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Besonders vorteilhaft erweisen sich die Eigenschaften des erfindungsgemäßen Mittels, wenn es als Aerosolspray, Aerosolschaum, Pumpspray oder Pumpschaum konfektioniert wird. Diese bevorzugte Form der Konfektionierung wird später im Detail beschrieben.

Stärke ist ein Reservekohlenhydrat, das von vielen Pflanzen in Form von üblicherweise 1 bis 200 $\mu$m großen Stärkekörnern (Granula) in verschiedenen Pflanzenteilen gespeichert wird, z.B. in Knollen oder Wurzeln, Getreidesamen, Früchten sowie im Mark. Eine erfindungsgemäß verwendbare nichtionische, mittels Propylenoxid modifizierte Stärke kann sich aus Stärke von Kartoffeln, Mais, Reis, Erbsen, Eicheln, Kastanien, Gerste, Weizen, Bananen, Sago, Hirse, Sorghum, Hafer, Gerste, Roggen, Bohnen, Batate, Maranta oder Maniok ableiten. Besonders ausgeprägte erfindungsgemäße Effekte werden durch nichtionische, mittels Propylenoxid modifizierte Tapioka Stärke bzw. nichtionische, mittels Propylenoxid modifizierte Kartoffelstärke bzw. durch Gemische beider vorgenannter Stärken, erzielt. Ganz besonders bevorzugt enthält das erfindungsgemäße Mittel mindestens eine nichtionische, mittels Propylenoxid modifizierte Kartoffelstärke.

Stärke gehört zu der Familie der Homoglycane und ist ein Polykondensationsprodukt von D-Glucose. Dabei besteht Stärke aus drei strukturell verschiedenen Polymeren der d-Glucopyranose, nämlich der Amylose, dem Amylopektin und einer sogenannten Zwischenfraktion. Höhere Pflanzen enthalten 0 bis 45 Gew.-% Amylose bezogen auf die Trockensubstanz.

Die Zwischenfraktion, die auch als anormales Amylopektin bezeichnet wird, steht strukturell zwischen der Amylose und dem Amylopektin. Die im Rahmen dieser Anmeldung definierten Mengenangaben für Amylopektin umfassen die Zwischenfraktion.

Es ist erfindungsgemäß bevorzugt, wenn die nichtionische, mittels Propylenoxid modifizierte Stärke einen Amylosegehalt von kleiner 25 Gew.-%, insbesondere von kleiner 20 Gew.-%, - jeweils bezogen auf das Gewischt der modifizierten Stärke - besitzt. Es zeigte sich, dass sich zur Erreichung des erfindungsgemäßen Effektes besonders eine Stärke eignet, die 17 bis 22 Gew.-% Amylose und 78 bis 83 Gew.-% Amylopektin enthält.

Amylose besteht aus überwiegend linear $\alpha$-1,4-glycosidisch verknüpfter d-Glucose, $M_r$ 50000-150000. Die resultierenden Ketten bilden in der Stärke Doppelhelices.

Amylopektin enthält neben den für Amylose beschriebenen $\alpha$-1,4-Verknüpfungen auch in einer Menge von 4 bis 6% $\alpha$-1,6-Bindungen als Verzweigungsstellen. Der durchschnittliche Abstand zwischen den Verzweigungsstellen beträgt etwa 12 bis 17 Glucose-Einheiten. Die Molmasse von $10^7$ bis $7 \cdot 10^8$ entspricht ca. $10^5$ Glucose-Einheiten, womit Amylopektin zu den größten Biopolymeren gehört. Besagte Verzweigungen sind derart über das Molekül verteilt, daß sich eine Büschelstruktur mit relativ kurzen Seitenketten entwickelt. Zwei dieser Seitenketten bilden jeweils eine Doppelhelix. Durch die vielen Verzweigungsstellen ist Amylopektin in Wasser relativ gut löslich.

Unter einer nichtionischen, mittels Propylenoxid modifizierten Stärke wird erfindungsgemäß ein Reaktionsprodukt aus einer Stärke und Propylenoxid verstanden. Ein solches Reaktionsprodukt umfasst mindestens eine Struktureinheit der Formel (PS),

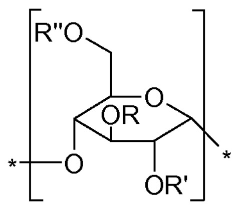

(PS),

worin mindestens ein Rest R, R' oder R" für eine Gruppe der Formel

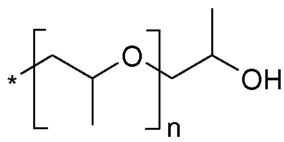

mit n ≥0 steht

und maximal 2 der Reste aus R, R', R" für ein Wasserstoffatom steht. Die nichtionischen, mittels Propylenoxid modifizierten Stärken werden beispielsweise durch Umsetzung einer nativen Stärke mit Propylenoxid bereitgestellt. Vor der Modifikation mit Propylenoxid kann die Stärke diversen physikalischen oder chemischen Prozessen ausgesetzt worden sein, wie z.B. einer Wärmebehandlung, Scherung, einer thermischen, säurehydrolytischen, oxidativen oder enzymatischen Spaltung, etc.

Es ist erfindungsgemäß bevorzugt, wenn die nichtionische, mittels Propylenoxid modifizierte Stärke in dem erfindungsgemäßen Mittel nicht in Form einzelner Stärkekörnchen (Granula) vorliegt. Zu diesem Zweck werden die Stärkekörner aufgeschlossen z.B. durch Hitze oder Scherung und die entsprechenden Polysaccharidmoleküle aus dem Verbund freigesetzt. Die freigesetzten Polysaccharidmoleküle werden nach oder vor der Freisetzung mittels Propylenoxid modifiziert.

Im Rahmen einer bevorzugten Ausführungsform ist die nichtionische, mittels Propylenoxid modifizierte Stärke verkleistert. Wird eine wässrige Suspension von Stärke erhitzt oder komprimiert, so beobachtet man bei einer kritischen Temperatur bzw. Druck eine tangentiale Quellung der Körper mit Verlust der Doppelbrechung, Änderung der Röntgenstruktur und abrupter Steigerung der Viskosität der Lösung. Diese Erscheinung wird Verkleisterung genannt.

Die erfindungsgemäßen nichtionischen, mittels Propylenoxid modifizierten Stärken liegen in dem erfindungsgemäßen Mittel in einer Molekulargewichtsverteilung vor. Erfindungsgemässe nichtionische, mittels Propylenoxid modifizierte Stärken weisen ein mittleres Molekulargewicht von 700 bis 1000 kDa (Gewichtsmittel) auf. Die Molekulargewichtsverteilung wurde experimentell mittels Gelfiltrationschromatographie gegen Dextran bestimmt. Das besagte Gewichtsmittel ist ein mittleres Molekulargewicht, welches das Totalgewicht der Moleküle verschiedenen Molekulargewichts berücksichtigt und nicht lediglich nur die Anzahl der Moleküle. Zur statistischen Berechnung des Gewichtsmittels wird zunächst der "Gewichtsbruch"

$$w_i = (N_i M_i) / [\Sigma(N_i M_i)]$$

definiert. Dieser gibt den Gewichtsanteil an Makromolekülen in der Probe an, die aus *i* Segmenten (z. B. Monomerbausteinen) der Masse $M_i$ bestehen und in der Probe $N_i$-mal vorkommen. Für das Gewichtsmittel des Molekulargewichts $M_w = \Sigma w_i M_i$ gilt damit

$$M_w = [\Sigma(N_i M^2_i)] / [\Sigma(N_i M_i)].$$

Zur Einstellung des Molekulargewichts wird die Stärke vor oder nach der Modifizierung mittels Propylenoxid einer mechanischen und/oder einer chemischen Behandlung unterzogen. Zur Molekulargewichtserhöhung kann die besagte Stärke vernetzt werden. Eine Vernetzung der nichtionischen, mittels Propylenoxid modifizierten Stärke liegt vor, wenn die linearen bzw. verzweigten Polysaccharid-Makromoleküle der Stärke kovalent durch ein Vernetzungsmittel unter Bildung eines dreidimensionalen, unlöslichen und nur noch quellbaren polymeren Netzwerkes verknüpft werden. Native Stärke gilt im Allgemeinen als unvernetzt und bedarf - falls eine Vernetzung gewünscht wäre - einer künstlichen Vernetzung mittels Synthesechemie. Eine solche künstliche Vernetzung lässt sich mit Vernetzungsmitteln durchführen.

(Nichtionische, mittels Propylenoxid modifizierte) Stärken, die eine solche Vernetzung nicht aufweisen, sind unvernetzt. Eine Vernetzung erfolgt beispielsweise durch das Vernetzungmittel Epichlorhydrin. Hierzu wird eine 42 Gew.-%-ige Mischung von nichtionischer, mittels Propylenoxid modifizierter Stärke in Wasser hergestellt, in diese die gewünschte Menge Epichlorhydrin bei Raumtemperatur eingerührt und nach einer Rührzeit von 1 bis 5 Stunden unter Kontrolle der Viskosität nach erreichen der Zielviskosität die vernetzte Stärke nach gängigen Verfahren isoliert.

Es ist jedoch erfindungsgemäß besonders bevorzugt, wenn die erfindungsgemäßen Mittel mindestens eine unvernetzte, nichtionische, mittels Propylenoxid modifizierte Stärke enthalten. Zur Erzielung eines niedrigeren Molekulargewichts von 100 bis 400 kDa, werden besagte Stärken bevorzugt einer mechanischen Spaltung, einer enzymatischen Spaltung (insbesondere mit alpha-Amylase, beta-Amylase, Glucoamylase oder entzweigende Enzyme), einer säurehydrolytischen Spaltung (insbesondere mit Salzsäure, Schwefelsäure oder Phosphorsäure), einer thermischen Spaltung oder einer Umsetzung mit Oxidationsmitteln (wie Periodat, Hypochlorit, Chromsäure, Permanganat, Stickstoffdioxid, Wasserstoffperoxid oder organischer Percarbonsäure, bevorzugt mit Wasserstoffperoxid), ausgesetzt. Zur mechanischen Spaltung der Stärke eignen sich Kneter, extruder, Stator/Rotor-Maschinen und/oder Rührwerke.

Die oxidative Spaltung mittels Wasserstoffperoxid ist erfindungsgemäß bevorzugt. Zu diesem Zweck wird beispielsweise die nichtionische, mittels Propylenoxid modifizierte Stärke in Wasser gegeben, auf 50 bis 70 °C erhitzt, Wasserstoffperoxid zugefügt und bei 70 bis 85°C für 2 bis 5 Stunden gerührt.

Der Gehalt an Propylenoxid der Stärke wirkt sich auf die Feinabstimmung des Frisurenhalts mit der Frisurenflexibilität und der Stabilität der kosmetischen Mittel aus. Es zeigte sich, dass die Parameter weiter optimiert werden, wenn die nichtionische, mittels Propylenoxid modifizierte Stärke - bezogen auf das Gewicht der modifizierten Stärke - einen Propylenoxidgehalt von 1 bis 20 Gew.-%, besonders bevorzugt einen Propylenoxidgehalt von 4 bis 12 Gew.-%, ganz besonders bevorzugt einen Propylenoxidgehalt von 9,5 bis 10,5 Gew.-% oder von 4,0 bis 6,0 Gew.-%, aufweist. Der Propylenoxidgehalt lässt sich beispielsweise nach Durchführung einer Hodges-Spaltung mit der Methode gemäß DIN EN 13268 bestimmen.

Ferner hat es sich erwiesen, dass sich solche kosmetischen Mittel im Sinne der Erfindung hervorragend eignen, in denen die nichtionische, mittels Propylenoxid modifizierte Stärke in einer 43 Gew.-%-igen wässrigen Lösung eine bevorzugte Viskosität im Bereich von 150 bis 1500000 mPa·s (Brookfield Viskosimeter, Spindel #7 bei 20°C und 20 U/min) aufweist. Besonders geeignete mittels Propylenoxid modifizierte Stärken weisen Viskositäten von 10000 bis 200000 mPa·s, besonders bevorzugt von 25000 bis 180000 mPa·s, (jeweils gemessen unter den zuvor genannten Bedingungen) auf.

Eine erfindungsgemäß besonders bevorzugte nichtionische, mittels Propylenoxid modifizierte Stärke ist unvernetzt, weist ein mittleres Molekulargewicht (Gewichtsmittel) von 700 bis 1000 kDa, auf und hat - bezogen auf das Gewicht der modifizierten Stärke - einen Propylenoxidgehalt von 1 bis 20 Gew.-%, besonders bevorzugt einen Propylenoxidgehalt von 4 bis 12 Gew.-%, ganz besonders bevorzugt einen Propylenoxidgehalt von 9,5 bis 10,5 Gew.-% oder von 4,0 bis 6,0 Gew.-%. Dabei handelt es sich wiederum bevorzugt um Tapiokastärke oder Kartoffelstärke, insbesondere um Kartoffelstärke.

Eine erfindungsgemäß besonders bevorzugte nichtionische, mittels Propylenoxid modifizierte Stärke ist unvernetzt, weist ein mittleres Molekulargewicht (Gewichtsmittel) von 700 bis 1000 kDa, auf und hat - bezogen auf das Gewicht der modifizierten Stärke - einen Propylenoxidgehalt von 1 bis 20 Gew.-%, besonders bevorzugt einen Propylenoxidgehalt von 4 bis 12 Gew.-%, ganz besonders bevorzugt einen Propylenoxidgehalt von 9,5 bis 10,5 Gew.-% oder von 4,0 bis 6,0 Gew.-%. Dabei handelt es sich wiederum bevorzugt um Tapiokastärke oder Kartoffelstärke, insbesondere um Kartoffelstärke.

Eine erfindungsgemäß ganz besonders bevorzugte nichtionische, mittels Propylenoxid modifizierte Kartoffelstärke ist unvernetzt, weist ein mittleres Molekulargewicht (Gewichtsmittel) von 700 bis 1000 kDa, auf und hat - bezogen auf das Gewicht der modifizierten Kartoffelstärke - einen Propylenoxidgehalt von 4 bis 12 Gew.-%, ganz besonders bevorzugt einen Propylenoxidgehalt von 9,5 bis 10,5 Gew.-% oder von 4,0 bis 6,0 Gew.-%.

Es ist erfindungsgemäß bevorzugt, wenn das kosmetisches Mittel die nichtionische, mittels Propylenoxid modifizierte Stärke in einer Menge von 0,2 Gew.-% bis 5,0 Gew.-%, besonders bevorzugt von 1,0 bis 3,0 Gew.-%, jeweils bezogen auf das Gewicht des Mittels, enthält.

Zusätzlich enthält das erfindungsgemäße Mittel zwingend mindestens ein filmbildendes kationisches und/oder festigendes kationisches Polymer.

Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine $C_{1-4}$-Kohlenwasserstoffgruppe an eine aus Acrylsäure, Methacrylsäure oder deren Derivaten aufgebaute Polymerhauptkette gebunden sind, haben sich als besonders geeignet erwiesen.

Ein erfindungsgemäß bevorzugt geeignetes kationisches filmbildendes und/oder kationisches festigendes Polymer ist mindestens ein kationisches filmbildendes und/oder kationisches festigendes Polymer, das mindestens ein Strukturelement der Formel (M9) und zusätzlich mindestens ein Strukturelement der Formel (M10) enthält

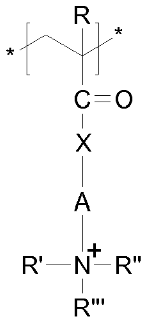
(M9)

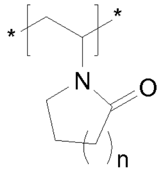
(M10)

worin

| | |
|---|---|
| R | für ein Wasserstoffatom oder eine Methylgruppe steht, |
| R', R" und R" | unabhängig voneinander stehen für eine ($C_1$ bis $C_{30}$)-Alkylgruppe, |
| X | steht für ein Sauerstoffatom oder eine Gruppe NH, |
| A | steht für eine Ethan-1,2,-diylgruppe oder eine Propan-1,3-diylgruppe, |
| n | 1 oder 3 bedeutet. |

Zur Kompensation der positiven Polymerladung dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Weiterhin eignen sich solche kationischen filmbildenden und/oder kationischen festigenden Polymere, die mindestens eine Struktureinheit der Formel (M5) und mindestens eine Struktureinheit der Formel (V) und gegebenenfalls mindestens eine Struktureinheit der Formel (VI) umfassen

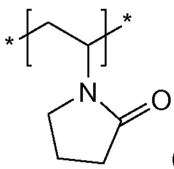
(M5)

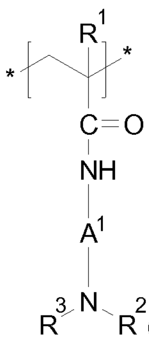
(V)

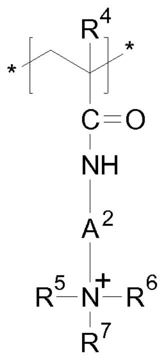
(VI)

worin

$R^1$ und $R^4$ stehen unabhängig voneinander für ein Wasserstoffatom oder eine Methylgruppe,
$A^1$ und $A^2$ stehen unabhängig voneinander für eine Gruppe Ethan-1,2-diyl, Propan-1,3-diyl oder Butan-1,4-diyl,
$R^2$, $R^3$, $R^5$ und $R^6$ stehen unabhängig voneinander für eine ($C_1$ bis $C_4$)-Alkylgruppe,
$R^7$ steht für eine ($C_8$ bis $C_{30}$)-Alkylgruppe.
Zur Kompensation der positiven Ladung des Monomers (VI) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Solche Verbindungen sind beispielsweise als

- Copolymere aus mit Diethylsulfat quaterniertem Dimethylaminoethylmethacrylat, mit Vinylpyrrolidon mit der INCI-Bezeichnung Polyquaternium-11 unter den Bezeichnungen Gafquat® 440, Gafquat®734, Gafquat®755 (jeweils Firma ISP) sowie Luviquat PQ 11 PN (Firma BASF SE),

- Copolymere aus N-Vinylpyrrolidon, N-(3-Dimethylaminopropyl)methacrylamid und 3-(Methacryloylamino)propyl-lauryl-dimethylammoniumchlorid (INCI-Bezeichnung:

  Polyquaternium-55), welches beispielsweise unter dem Handelsnamen Styleze W 10 oder Styleze W 20 (10 oder 20 Gew.-% Aktivsubstanz in Ethanol-Wasser-Gemisch) von der Firma ISP vertrieben wird.

- Copolymere aus N-Vinylpyrrolidon, N-Vinylcaprolactam, N-(3-Dimethylaminopropyl)methacrylamid und 3-(Methacryloylamino)propyl-lauryldimethylammoniumchlorid (INCI-Bezeichnung: Polyquaternium-69), welches beispielsweise unter dem Handelsnamen AquaStyle® 300 (28-32 Gew.-% Aktivsubstanz in Ethanol-Wasser-Gemisch) von der Firma ISP vertrieben wird.

Es gelten somit insbesondere solche Mittel im Rahmen dieser Ausführungsform als ganz besonders bevorzugt, die in einem kosmetisch akzeptablen Träger enthalten

(a) mindestens eine unvernetzte, nichtionische, mittels Propylenoxid modifizierte Stärke, insbesondere mit einem mittleren Molekulargewicht (Gewichtsmittel) von 50 bis 2500 kDa,
und
(b) mindestens ein kationisches filmbildendes und/oder kationisches festigendes Polymer das das mindestens ein Strukturelement der Formel (M9) und zusätzlich mindestens ein Strukturelement der Formel (M10) enthält

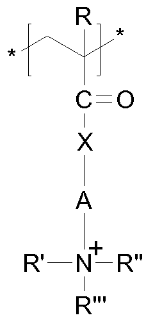
(M9)

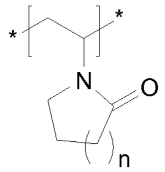
(M10)

worin

| | |
|---|---|
| R | für ein Wasserstoffatom oder eine Methylgruppe steht, |
| R', R" und R" | unabhängig voneinander stehen für eine ($C_1$ bis $C_{30}$)-Alkylgruppe, |
| X | steht für ein Sauerstoffatom oder eine Gruppe NH, |
| A | steht für eine Ethan-1,2,-diylgruppe oder eine Propan-1,3-diylgruppe, |
| n | 1 oder 3 bedeutet. |

Es gelten weiterhin *mutatis mutandis* die bevorzugten Stärken der Komponente (a) (*vide supra*) als bevorzugt.
Weiterhin werden die kationischen filmbildenden und/oder kationischen festigenden Polymere erfindungsgemäß besonders bevorzugt aus kationischen, quaternisierten Cellulose-Derivaten ausgewählt.
Ferner eignen sich als filmbildendes und/oder festigendes Polymere bevorzugt kationische, quaternisierte Cellulosederivate.
Es erweisen sich solche kationischen, quaternisierten Cellulosen als im Sinne der Erfindung besonders vorteilhaft, die in einer Seitenkette mehr als eine permanente kationische Ladung tragen. Unter diesen kationischen Cellulosen sind wiederum solche kationischen Cellulosen mit der INCI-Bezeichnung Polyquaternium-4 besonders geeignet, welche beispielsweise unter den Bezeichnungen Celquat® H 100, Celquat® L 200 von der Firma National Starch vertrieben werden.
Es gelten somit insbesondere solche Mittel im Rahmen dieser Ausführungsform als ganz besonders bevorzugt, die in einem kosmetisch akzeptablen Träger enthalten

(a) mindestens eine unvernetzte, nichtionische, mittels Propylenoxid modifizierte Stärke, insbesondere mit einem mittleren Molekulargewicht (Gewichtsmittel) von 50 bis 2500 kDa,
und
(b) Polyquaternium-4

Als im Sinne der Erfindung besonders bevorzugt verwendbare kationische Polymere dienen weiterhin solche kationischen filmbildenden und/oder kationischen festigenden Copolymere, die mindestens ein Strukturelement der Formel (M11)

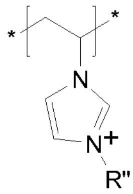

(M11)

worin R" für eine ($C_1$ bis $C_4$)-Alkylgruppe, insbesondere eine Methylgruppe, steht,
und zusätzlich mindestens ein weiteres kationisches und/oder nichtionisches Strukturelement aufweisen.

Zur Kompensation der positiven Polymerladung dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Es ist wiederum erfindungsgemäß bevorzugt, wenn als zusätzliches kationisches filmbildendes und/oder kationisches festigendes Polymer, mindestens ein Copolymer (co1) enthalten ist, das neben mindestens einem Strukturelement der Formel (M11) zusätzlich ein Strukturelement der Formel (M5) umfasst

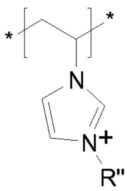

(M11)

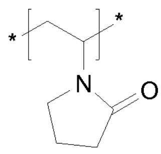

(M5).

worin R" für eine ($C_1$ bis $C_4$)-Alkylgruppe, insbesondere eine Methylgruppe, steht.

Zur Kompensation der positiven Polymerladung der Copolymere (co1) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Ganz besonders bevorzugte kationische filmbildende und/oder kationische festigende Polymere als Copolymere (co1) enthalten 10 bis 30 Mol-%, vorzugsweise 15 bis 25 Mol.-% und insbesondere 20 Mol.-% Struktureinheiten gemäß Formel (M11) und 70 bis 90 Mol.-%, vorzugsweise 75 bis 85 Mol.-% und insbesondere 80 Mol.-% Struktureinheiten gemäß Formel (M5).

Hierbei ist besonders bevorzugt, wenn die Copolymere (co1) neben Polymereinheiten, die aus dem Einbau der genannten Struktureinheiten gemäß Formel (M11) und (M5) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (co1) ausschließlich aus Struktureinheiten der Formel (M11) mit R" = Methyl und (M5) aufgebaut.

Wird in zur Kompensation der positiven Ladung des Copolymers ein Chloridion verwendet, so werden diese N-Methylvinylimidazol/Vinylpyrrolidon-Copolymere werden laut INCI-Nomenklatur als Polyquaternium-16 bezeichnet und sind beispielsweise von der BASF unter den Handelsnamen Luviquat® Style, Luviquat® FC 370, Luviquat® FC 550, Luviquat® FC 905 und Luviquat® HM 552

Wird in zur Kompensation der positiven Ladung des Copolymers ein Methosulfat verwendet, so werden diese N-Methylvinylimidazol/Vinylpyrrolidon-Copolymere werden laut INCI-Nomenklatur als Polyquaternium-44 bezeichnet und sind beispielsweise von der BASF unter den Handelsnamen Luviquat® UltraCare erhältlich.

Es gelten somit insbesondere solche Mittel im Rahmen dieser Ausführungsform als ganz besonders bevorzugt, die in einem kosmetisch akzeptablen Träger enthalten

(a) mindestens eine unvernetzte, nichtionische, mittels Propylenoxid modifizierte Stärke, insbesondere mit einem mittleren Molekulargewicht (Gewichtsmittel) von 50 bis 2500 kDa, und
(b) mindestens ein kationisches filmbildendes und/oder kationisches festigendes Polymer das neben mindestens einem Strukturelement der Formel (M11) zusätzlich ein Strukturelement der Formel (M5) umfasst

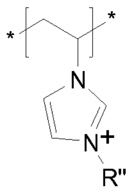 (M11) 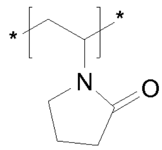 (M5).

worin R" für eine ($C_1$ bis $C_4$)-Alkylgruppe, insbesondere eine Methylgruppe, steht.

Es gelten weiterhin *mutatis mutandis* die bevorzugten Stärken der Komponente (a) (*vide supra*) als bevorzugt.

Zusätzlich zu dem bzw. den Copolymer(en) (co1) oder an dessen bzw. deren Stelle können die erfindungsgemäßen Mittel auch Copolymere (co2) enthalten, die ausgehend vom Copolymer (co1) als zusätzliche Struktureinheiten Struktureinheiten der Formel (M6) aufweisen

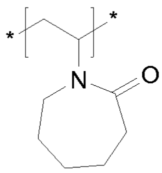 (M6).

Weitere besonders bevorzugte erfindungsgemäße Mittel sind somit dadurch gekennzeichnet, daß sie als kationisches filmbildendes und/oder kationisches festigendes Polymer mindestens ein Copolymer (co2) enthalten, das mindestens eine Struktureinheit gemäß Formel (M11-a) und mindestens eine Struktureinheit gemäß Formel (M5) und mindestens eine Struktureinheit gemäß Formel (M6) enthält

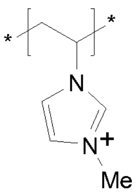 (M11-a) 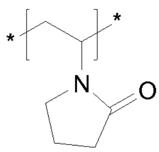 (M5) 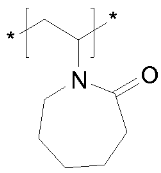 (M6)

Auch hierbei ist besonders bevorzugt, wenn die Copolymere (co2) neben Polymereinheiten, die aus dem Einbau der genannten Struktureinheiten gemäß Formel (M11-a), (M5) und (M6) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (co2) ausschließlich aus Struktureinheiten der Formeln (M11-a), (M5) und (M6) aufgebaut.

Zur Kompensation der positiven Polymerladung der Komponente (co2) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Copolymers ein Methosulfat verwendet werden solche N-Methylvinylimidazol/Vinylpyrrolidon/Vinylcaprolactam-Copolymere laut INCI-Nomenklatur als Polyquarternium-46 bezeichnet und sind beispielsweise von der BASF unter dem Handelsnamen Luviquat® Hold erhältlich.

Ganz besonders bevorzugte Copolymere (co2) enthalten 1 bis 20 Mol-%, vorzugsweise 5 bis 15 Mol.-% und insbesondere 10 Mol.-% Struktureinheiten gemäß Formel (M11-a) und 30 bis 50 Mol.-%, vorzugsweise 35 bis 45 Mol.-% und insbesondere 40 Mol.-% Struktureinheiten gemäß Formel (M5) und 40 bis 60 Mol.-%, vorzugsweise 45 bis 55 Mol.-% und insbesondere 60 Mol.-% Struktureinheiten gemäß Formel (M6).

Es gelten somit insbesondere solche Mittel im Rahmen dieser Ausführungsform als ganz besonders bevorzugt, die in einem kosmetisch akzeptablen Träger enthalten

(a) mindestens eine unvernetzte, nichtionische, mittels Propylenoxid modifizierte Stärke, insbesondere mit einem mittleren Molekulargewicht (Gewichtsmittel) von 50 bis 2500 kDa,
und
(b) mindestens ein kationisches filmbildendes und/oder kationisches festigendes Polymer das mindestens einem Strukturelement der Formel (M11), mindestens ein Strukturelement der Formel (M5) und mindestens ein Strukturelement der Formel (M6) umfasst

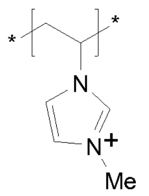 (M11-a) 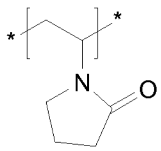 (M5) 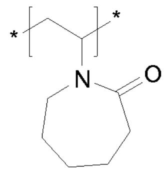 (M6).

Es gelten weiterhin *mutatis mutandis* die bevorzugten Stärken der Komponente (a) *(vide supra)* als bevorzugt.

Zusätzlich zu dem bzw. den Copolymer(en) (co1) und/oder (co2) oder an dessen bzw. deren Stelle können die erfindungsgemäßen Mittel als filmbildendes kationische und/oder festigendes kationisches Polymer auch Copolymere (co3) enthalten, die als Struktureinheiten Struktureinheiten der Formeln (M11-a) und (M5) aufweisen, sowie weitere Struktureinheiten aus der Gruppe der Vinylimidazol-Einheiten und weitere Struktureinheiten aus der Gruppe der Acrylamid- und/oder Methacrylamid-Einheiten.

Weitere besonders bevorzugte erfindungsgemäße Mittel sind dadurch gekennzeichnet, daß sie als zusätzliches kationisches filmbildendes und/oder kationisches festigendes Polymer mindestens ein Copolymer (co3) enthalten, das mindestens eine Struktureinheit gemäß Formel (M11-a) und mindestens eine Struktureinheit gemäß Formel (M5) und mindestens eine Struktureinheit gemäß Formel (M10) und mindestens eine Struktureinheit gemäß Formel (M12) enthält

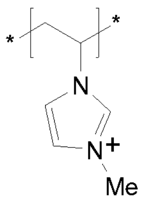 (M11-a) 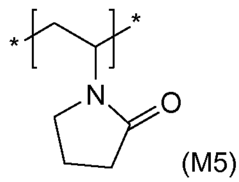 (M5) 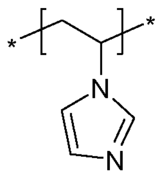 (M8) 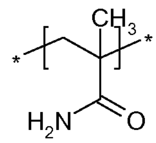 (M12).

Auch hierbei ist besonders bevorzugt, wenn die Copolymere (co3) neben Polymereinheiten, die aus dem Einbau der genannten Struktureinheiten gemäß Formel (M11-a), (M5), (M8) und (M12) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (co3) ausschließlich aus Struktureinheiten der Formel (M11-a), (M5), (M8) und (M12) aufgebaut.

Zur Kompensation der positiven Polymerladung der Komponente (co3) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Copolymers ein Methosulfat verwendet werden solche N-Methylvinylimidazol/Vinylpyrrolidon/Vinylimidazol/Methacrylamid-Copolymere laut INCI-Nomenklatur als Polyquaternium-68 bezeichnet und sind beispielsweise von der BASF unter dem Handelsnamen Luviquat® Supreme erhältlich.

Ganz besonders bevorzugte Copolymere (co3) enthalten 1 bis 12 Mol-%, vorzugsweise 3 bis 9 Mol.-% und insbesondere 6 Mol.-% Struktureinheiten gemäß Formel (M11-a) und 45 bis 65 Mol.-%, vorzugsweise 50 bis 60 Mol.-% und insbesondere 55 Mol.-% Struktureinheiten gemäß Formel (M5) und 1 bis 20 Mol.-%, vorzugsweise 5 bis 15 Mol.-% und insbesondere 10 Mol.-% Struktureinheiten gemäß Formel (M8) und 20 bis 40 Mol.-%, vorzugsweise 25 bis 35 Mol.-% und insbesondere 29 Mol.-% Struktureinheiten gemäß Formel (M12).

Es gelten somit insbesondere solche Mittel im Rahmen dieser Ausführungsform als ganz besonders bevorzugt, die in einem kosmetisch akzeptablen Träger enthalten

(a) mindestens eine unvernetzte, nichtionische, mittels Propylenoxid modifizierte Stärke, insbesondere mit einem mittleren Molekulargewicht (Gewichtsmittel) von 50 bis 2500 kDa,
und
(b) mindestens ein kationisches filmbildendes und/oder kationisches festigendes Polymer mindestens eine Struktureinheit gemäß Formel (M11-a) und mindestens eine Struktureinheit gemäß Formel (M5) und mindestens eine Struktureinheit gemäß Formel (M10) und mindestens eine Struktureinheit gemäß Formel (M12) enthält

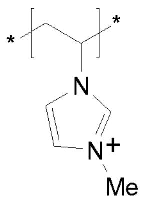 (M11-a) 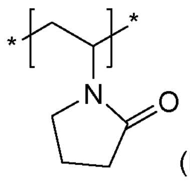 (M5) 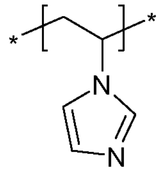 (M8) 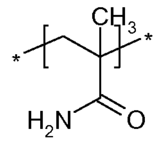 (M12).

Es gelten weiterhin *mutatis mutandis* die bevorzugten Stärken der Komponente (a) *(vide supra)* als bevorzugt.

Unter den zusätzlichen filmbildenden kationischen und/oder festigenden Polymer ausgewählt aus den kationischen Polymeren mit mindestens einem Strukturelement der obigen Formel (M11-a), gelten als bevorzugt:

- Vinylpyrrolidon/1-Vinyl-3-methyl-1H-imidazoliumchlorid-Copolymere (wie beispielsweise das mit der INCI-Bezeichnung Polyquaternium-16 unter den Handelsbezeichnungen Luviquat® Style, Luviquat® FC 370, Luviquat® FC 550, Luviquat® FC 905 und Luviquat® HM 552 (BASF SE)),
- Vinylpyrrolidon/1-Vinyl-3-methyl-1H-imidazoliummethylsulfat-Copolymere (wie beispielsweise das mit der INCI-Bezeichnung Polyquaternium-44 unter den Handelsbezeichnungen Luviquat® Care (BASF SE)),
- Vinylpyrrolidon/Vinylcaprolactam/1-Vinyl-3-methyl-1H-imidazolium-Terpolymer (wie beispielsweise das mit der INCI-Bezeichnung Polyquaternium-46 unter den Handelsbezeichnungen Luviquat® Care oder Luviquat® Hold (BASF SE)),
- Vinylpyrrolidon/Methacrylamid/Vinylimidazol/1-Vinyl-3-methyl-1H-imidazoliummethylsulfat-Copolymer (wie beispielsweise das mit der INCI-Bezeichnung Polyquaternium-68 unter der Handelsbezeichnung Luviquat® Supreme (BASF SE)),

sowie Gemische aus diesen Polymeren.

Weitere in den erfindungsgemäßen Mitteln bevorzugt einsetzbare kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt.

Als im Sinne der Erfindung bevorzugt geeignete temporär kationische Polymere gelten gleichfalls solche, die mindestens eine Struktureinheit der Formeln (M1-1) bis (M1-8) aufweisen

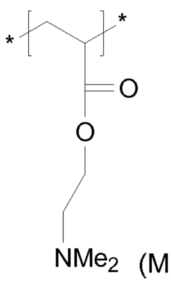 (M1-1) 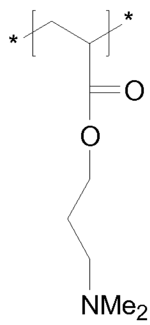 (M1-2) 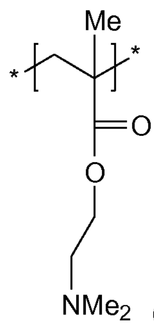 (M1-3) 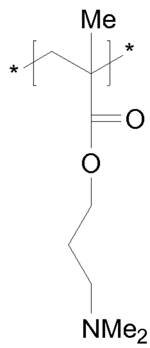 (M1-4)

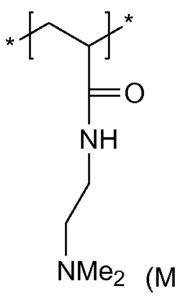 (M1-5) 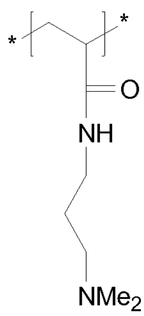 (M1-6) 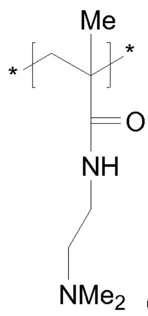 (M1-7) 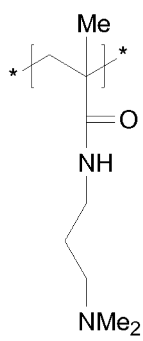 (M1-8).

Dabei sind wiederum solche Copolymere bevorzugt, die mindestens eine Struktureinheit der Formeln (M1-1) bis (M1-8) und zusätzlich mindestens eine Struktureinheit der Formel (M10) enthalten,

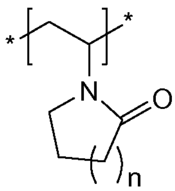

(M10)

worin

n 1 oder 3 bedeutet.

Dabei gilt wiederum die Gruppe der Polymere

- Vinylcaprolactam/Vinylpyrrolidon/Dimethylaminoethylmethacrylat-Copolymer (beispielsweise INCI-Bezeichnung: Vinyl Caprolactam/PVP/Di-methylaminoethyl Methacrylate Copolymer unter dem Handelsnamen Gaffix® VC 713 (ISP)),
- Vinylpyrrolidon/Vinylcaprolactam/Dimethylaminopropylmethacrylamid-Copolymer (beispielsweise INCI-Bezeichnung: VP/Vinyl Caprolactam/DMAPA Acrylates Copolymer unter dem Handelsnamen Aquaflex® SF-40 (ISP)),
- Vinylcaprolactam/Vinylpyrrolidon/Dimethylaminoethylmethacrylat-Copolymer (beispielsweise als.35-39% Festkörper in Ethanol in form des Handelsprodukts Advantage LC E mit der INCI-Bezeichnung: Vinyl Caprolactam/VP/Dimethylaminoethyl Methacrylate Copolymer, Alcohol, Lauryl Pyrrolidone (ISP)),
- Vinylpyrrolidon/Dimethylaminopropylmethacrylamid-Copolymer (beispielsweise INCI-Bezeichnung: VP/DMAPA Acrylates Copolymer unter dem Handelsnamen Styleze CC-10 (10 Gew.-% Aktivsubstanz) (ISP)),

als bevorzugte Liste zur Auswahl mindestens eines oder mehrerer Polymere daraus.

Weiterhin ist es erfindungsgemäß besonders bevorzugt, wenn neben der besagten Stärke (a) und dem filmbildenden, kationischen und/oder festigenden, kationischen Polymer (b) zusätzlich mindestens ein filmbildendes nichtionisches und/oder festigendes, nichtionisches Polymer (c) enthalten ist. Dieses Polymer ist von der nichtionischen, mittels Propylenoxid modifizierten Stärke verschieden. Unter einem nichtionischen Polymer wird erfindungsgemäß ein Polymer verstanden, das in einem protischen Lösemittel bei Standardbedingungen im Wesentlichen keine Struktureinheiten mit kationischen oder anionischen Gruppen trägt, welche durch Gegenionen unter Erhaltung der Elektroneutralität kompensiert werden müssen.

Die filmbildenden nichtionischen und/oder festigenden nichtionischen Polymere (c) sind in dem erfindungsgemäßen Mittel bevorzugt in einer Menge von 0,1 Gew.-% bis 20,0 Gew.-%, besonders bevorzugt von 0,2 Gew.-% bis 15,0 Gew.-%, ganz besonders bevorzugt von 0,5 Gew.-% bis 10,0 Gew.-%, jeweils bezogen auf das Gewicht des erfindungsgemäßen Mittels, enthalten. Bevorzugte nichtionische filmbildende und/oder nichtionische haarfestigende Polymere (c) enthalten mindestens eine Struktureinheit, ausgewählt aus der Gruppe der Struktureinheiten der Formeln (M1) bis (M6)

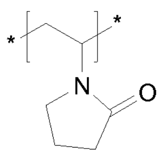 (M5) 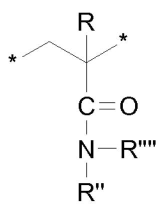 (M2) 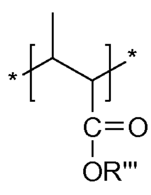 (M3)

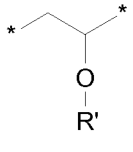 (M4) 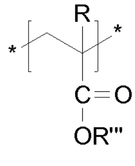 (M7) 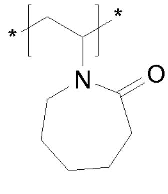 (M6)

worin

R steht für ein Wasserstoffatom oder eine Methylgruppe,

R’ steht für ein Wasserstoffatom oder eine ($C_1$ bis $C_4$)-Acylgruppe,

R" und R""  stehen unabhängig voneinander für eine ($C_1$ bis $C_7$)-Alkylgruppe oder ein Wasserstoffatom
R"'  steht für eine lineare oder verzweigte ($C_1$ bis $C_4$)-Alkylgruppe oder eine ($C_2$ bis $C_4$)-Hydroxyalkylgruppe.

Besonders bevorzugte, nichtionische filmbildende und/oder nichtionische haarfestigende Polymere (c) sind Homo-oder Copolymere, die aus mindestens einem der folgenden Monomere aufgebaut sind: N-Vinylpyrrolidon, N-Vinylcaprolactam, Vinylester (wie z.B. Vinylacetat, Vinylalkohol), Acrylamid, Methacrylamid, Alkyl- und Dialkylacrylamid (insbesondere N-Methyl- und N,N-Dimethylacrylamid), Alkyl- und Dialkylmethacrylamid (insbesondere N-Methyl- und N,N-Dimethylmethacrylamid), Alkylacrylat, Alkylmethacrylat, wobei jeweils die Alkylgruppen dieser Monomere aus ($C_1$ bis $C_3$)-Alkylgruppen ausgewählt werden.
Für die erfindungsgemäßen Mittel ganz besonders geeignete nichtionische Polymere auf Basis ethylenisch ungesättigter Monomere enthalten mindestens eine der nachfolgenden Struktureinheiten

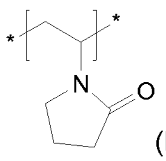
(M5)
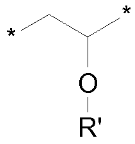
(M4)
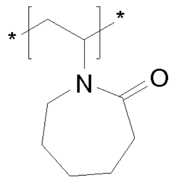
(M6)

worin R' steht für ein Wasserstoffatom oder eine ($C_1$- bis $C_{30}$)-Acylgruppe, insbesondere für ein Wasserstoffatom oder eine Acetylgruppe.
Geeignet sind insbesondere Homopolymere des Vinylcaprolactams oder des Vinylpyrrolidons (wie beispielsweise Luviskol® K 90 oder Luviskol® K 85 der Firma BASF SE), Copolymerisate aus Vinylpyrrolidon und Vinylacetat (Dabei ist es wiederum bevorzugt, wenn das Molverhältnis der aus dem Monomer N-Vinylpyrrolidon enthaltenen Struktureinheiten zu den aus dem Monomer Vinylacetat enthaltenen Struktureinheiten des Polymers im Bereich von 20 zu 80 bis 80 zu 20, insbesondere von 30 zu 70 bis 60 zu 40, liegt; beispielsweise vertrieben unter dem Warenzeichen Luviskol® VA 37, Luviskol® VA 55, Luviskol® VA 64 und Luviskol® VA 73 von der Firme BASF SE), Terpolymere aus Vinylpyrrolidon, Vinylacetat und Vinylpropionat, Polyacrylamide (wie beispielsweise Akypomine P 191 von der Firma CHEM-Y), Polyvinylalkohole (die beispielsweise unter den Handelsbezeichnungen Elvanol® von Du Pont oder Vinol® 523/540 von der Firma Air Products vertrieben werden), Terpolymere aus Vinylpyrrolidon, Methacrylamid und Vinylimidazol (wie beispielsweise Luviset® Clear der Firma BASF SE).
Mittel, die als filmbildendes nichtionisches und/oder festigendes nichtionisches Polymer (c) mindestens ein Polymer ausgewählt aus der Gruppe, die gebildet wird aus

- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,

sind erfindungsgemäß ganz besonders bevorzugt.
Es gelten weiterhin insbesondere solche Mittel als ganz besonders bevorzugte Ausführungsformen der Variante mit zusätzlichem filmbildenden nichtionischen und/oder festigenden nichtionischen Polymer (c):
Kosmetisches Mittel, enthaltend in einem kosmetisch akzeptablen Träger

(a) mindestens eine unvernetzte, nichtionische, mittels Propylenoxid modifizierte Kartoffelstärke mit einem Molekulargewicht (Gewichtsmittel) von 700 bis 1000, und
(b) mindestens ein filmbildendes kationisches und/oder festigendes kationisches Polymer, und
(c) Polyvinylpyrrolidon.

Kosmetisches Mittel, enthaltend in einem kosmetisch akzeptablen Träger

(a) mindestens eine unvernetzte, nichtionische, mittels Propylenoxid modifizierte Kartoffelstärke mit einem Molekulargewicht (Gewichtsmittel) von 700 bis 1000, und
(b) mindestens ein filmbildendes kationisches und/oder festigendes kationisches Polymer, und
(c) ein Copolymer, welches aus den Monomeren N-Vinylpyrrolidon und Vinylacetat - insbesondere aus keinem weiteren Monomeren - hergestellt wird.

Kosmetisches Mittel, enthaltend in einem kosmetisch akzeptablen Träger

(a) mindestens eine unvernetzte, nichtionische, mittels Propylenoxid modifizierte Stärke mit einem Molekulargewicht (Gewichtsmittel) von 700 bis 1000, und mit einem Propylenoxidgehalt von 1 bis 20 Gew.-%, bevorzugt einem Propylenoxidgehalt von 4 bis 12 Gew.-%, besonders bevorzugt einem Propylenoxidgehalt von 9,5 bis 10,5 Gew.-% oder von 4,0 bis 6,0 Gew.-%, jeweils bezogen auf das Gewicht der modifizierten Stärke, und
(b) mindestens ein filmbildendes kationisches und/oder festigendes kationisches Polymer, und
(c) Polyvinylpyrrolidon.

Kosmetisches Mittel, enthaltend in einem kosmetisch akzeptablen Träger

(a) mindestens eine unvernetzte, nichtionische, mittels Propylenoxid modifizierte Stärke mit einem Molekulargewicht (Gewichtsmittel) 700 bis 1000, und mit einem Propylenoxidgehalt von 1 bis 20 Gew.-%, bevorzugt einem Propylenoxidgehalt von 4 bis 12 Gew.-%, besonders bevorzugt einem Propylenoxidgehalt von 9,5 bis 10,5 Gew.-% oder von 4,0 bis 6,0 Gew.-%, jeweils bezogen auf das Gewicht der modifizierten Stärke, und
(b) mindestens ein filmbildendes kationisches und/oder festigendes kationisches Polymer, und
(c) ein Copolymer, welches aus den Monomeren N-Vinylpyrrolidon und Vinylacetat - insbesondere aus keinem weiteren Monomeren - hergestellt wird.

Kosmetisches Mittel, enthaltend in einem kosmetisch akzeptablen Träger

(a) mindestens eine unvernetzte, nichtionische, mittels Propylenoxid modifizierte Kartoffelstärke mit einem Molekulargewicht (Gewichtsmittel) von 700 bis 1000, und mit einem Propylenoxidgehalt von 1 bis 20 Gew.-%, bevorzugt einem Propylenoxidgehalt von 4 bis 12 Gew.-%, besonders bevorzugt einem Propylenoxidgehalt von 9,5 bis 10,5 Gew.-% oder von 4,0 bis 6,0 Gew.-%, jeweils bezogen auf das Gewicht der modifizierten Stärke, und
(b) mindestens ein filmbildendes kationisches und/oder festigendes kationisches Polymer, und
(c) Polyvinylpyrrolidon.

Kosmetisches Mittel, enthaltend in einem kosmetisch akzeptablen Träger

(a) mindestens eine unvernetzte, nichtionische, mittels Propylenoxid modifizierte Kartoffelstärke mit einem Molekulargewicht (Gewichtsmittel) von 700 bis 1000, und mit einem Propylenoxidgehalt von 1 bis 20 Gew.-%, bevorzugt einem Propylenoxidgehalt von 4 bis 12 Gew.-%, besonders bevorzugt einem Propylenoxidgehalt von 9,5 bis 10,5 Gew.-% oder von 4,0 bis 6,0 Gew.-%, jeweils bezogen auf das Gewicht der modifizierten Stärke, und
(b) mindestens ein filmbildendes kationisches und/oder festigendes kationisches Polymer, und
(c) ein Copolymer, welches aus den Monomeren N-Vinylpyrrolidon und Vinylacetat - insbesondere aus keinem weiteren Monomeren - hergestellt wird.

Kosmetisches Mittel, enthaltend in einem kosmetisch akzeptablen Träger

(a) mindestens eine unvernetzte, nichtionische, mittels Propylenoxid modifizierte Stärke mit einem Molekulargewicht (Gewichtsmittel) von 700 bis 1000, und mit einem Propylenoxidgehalt von 4 bis 12 Gew.-%, besonders bevorzugt einem Propylenoxidgehalt von 9,5 bis 10,5 Gew.-% oder von 4,0 bis 6,0 Gew.-%, jeweils bezogen auf das Gewicht der modifizierten Stärke, und
(b) mindestens ein filmbildendes kationisches und/oder festigendes kationisches Polymer, und
(c) Polyvinylpyrrolidon.

Kosmetisches Mittel, enthaltend in einem kosmetisch akzeptablen Träger

(a) mindestens eine unvernetzte, nichtionische, mittels Propylenoxid modifizierte Stärke mit einem Molekulargewicht (Gewichtsmittel) von 700 bis 1000, und mit einem Propylenoxidgehalt von 4 bis 12 Gew.-%, besonders bevorzugt einem Propylenoxidgehalt von 9,5 bis 10,5 Gew.-% oder von 4,0 bis 6,0 Gew.-%, jeweils bezogen auf das Gewicht der modifizierten Stärke, und
(b) mindestens ein filmbildendes kationisches und/oder festigendes kationisches Polymer, und
(c) ein Copolymer, welches aus den Monomeren N-Vinylpyrrolidon und Vinylacetat - insbesondere aus keinem weiteren Monomeren - hergestellt wird.

Kosmetisches Mittel, enthaltend in einem kosmetisch akzeptablen Träger

(a) mindestens eine unvernetzte, nichtionische, mittels Propylenoxid modifizierte Kartoffelstärke mit einem Molekulargewicht (Gewichtsmittel) von 700 bis 1000, und mit einem Propylenoxidgehalt von 4 bis 12 Gew.-%, besonders bevorzugt einem Propylenoxidgehalt von 9,5 bis 10,5 Gew.-% oder von 4,0 bis 6,0 Gew.-%, jeweils bezogen auf das Gewicht der modifizierten Stärke, und
(b) mindestens ein filmbildendes kationisches und/oder festigendes kationisches Polymer, und
(c) Polyvinylpyrrolidon.

Kosmetisches Mittel, enthaltend in einem kosmetisch akzeptablen Träger

(a) mindestens eine unvernetzte, nichtionische, mittels Propylenoxid modifizierte Kartoffelstärke mit einem Molekulargewicht (Gewichtsmittel) von 700 bis 1000, und mit einem Propylenoxidgehalt von 4 bis 12 Gew.-%, besonders bevorzugt einem Propylenoxidgehalt von 9,5 bis 10,5 Gew.-% oder von 4,0 bis 6,0 Gew.-%, jeweils bezogen auf das Gewicht der modifizierten Stärke, und
(b) mindestens ein filmbildendes kationisches und/oder festigendes kationisches Polymer, und
(c) ein Copolymer, welches aus den Monomeren N-Vinylpyrrolidon und Vinylacetat - insbesondere aus keinem weiteren Monomeren - hergestellt wird.

Im Rahmen der Ausführungsformen gelten die bevorzugten Ausführungsformen, insbesondere die bevorzugten Einsatzmengen der besagten Komponenten. Auch die bevorzugten Viskositäten der besagten mittels Propylenoxid modifizierten Stärke gelten gemäß Ausführungsformen als bevorzugt. Die als bevorzugt genannten filmbildenden kationischen und/oder festigenden kationischen Polymere (*vide supra*) gelten gemäß Ausführungsformen ebenso als bevorzugt.
Zur Intensivierung des erfindungsgemäßen Effektes enthalten die erfindungsgemäßen Mittel vorzugsweise zusätzlich mindestens ein Tensid, wobei sich prinzipiell nichtionische, anionische, kationische, ampholytische Tenside eignen. Die Gruppe der ampholytischen oder auch amphoteren Tenside umfasst zwitterionische Tenside und Ampholyte. Die Tenside können erfindungsgemäß bereits emulgierende Wirkung haben.
Die zusätzlichen Tenside sind in dem erfindungsgemäß Mittel bevorzugt in einer Menge von 0,01 Gew.-% bis 5 Gew.-%, besonders bevorzugt von 0,05 Gew.-% bis 0,5 Gew.-%, jeweils bezogen auf das Gewicht des Mittels, enthalten.
Es hat sich als besonders bevorzugt erwiesen, wenn die erfindungsgemäßen Mittel zusätzlich mindestens ein nichtionisches Tensid enthalten.
Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 100 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder $C_2$ - $C_6$ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- $C_{12}$-$C_{30}$-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (E4-I)

$$R^1CO\text{-}(OCH_2CHR_2)_wOR^3 \qquad \text{(E4-I)}$$

  in der $R^1CO$ für einen linearen oder verzweigten, gesättigten und/oder ungesättigten Acylrest mit 6 bis 22 Kohlenstoffatomen, $R^2$ für Wasserstoff oder Methyl, $R^3$ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,

- Zuckertenside vom Typ der Alkyl- und Alkenyloligoglykoside gemäß Formel (E4-II),

$R^{4O}$-$[G]_p$ (E4-II)

in der $R^4$für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.
Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Als ganz besonders bevorzugte nichtionische Tenside haben sich die Alkylenoxid-Anlagerungsprodukte an gesättigte lineare Fettalkohole und Fettsäuren mit jeweils 2 bis 100 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside $C_{12}$-$C_{30}$-Fettsäuremono- und -diester von Anlagerungsprodukten von 1 bis 30 Mol Ethylenoxid an Glycerin und/oder Anlagerungsprodukte von 5 bis 60 Mol Ethylenoxid an Rizinusöl und gehärtetes Rizinusöl enthalten.
Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.
Ganz besonders bevorzugt enthalten die erfindungsgemäßen Mittel als Tensid mindestens ein Anlagerungsprodukt von 15 bis 100 mol Ethylenoxid, insbesondere von 15 bis 50 mol Ethylenoxid an einen linearen oder verzweigten (insbesondere linearen) Fettalkohol mit 8 bis 22 Kohlenstoffatomen. Es handelt sich dabei ganz besonders bevorzugt um Ceteareth-15, Ceteareth-25 oder Ceteareth-50, welche als Eumulgin® CS 15 (COGNIS), Cremophor A25 (BASF SE) bzw. Eumulgin® CS 50 (COGNIS) vermarktet werden.
Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,

- lineare und verzweigte Fettsäuren mit 8 bis 30 C-Atomen (Seifen),
- Ethercarbonsäuren der Formel R-O-$(CH_2$-$CH_2O)_x$-$CH_2$-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O$(CH_2$-$CH_2O)_x$-$OSO_3H$, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,

- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I),

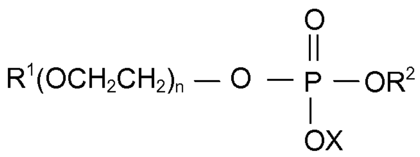
(E1-I)

in der $R^1$ bevorzugt für einen aliphatischen Kohlenwasserstoffrest mit 8 bis 30 Kohlenstoffatomen, $R^2$ für Wasserstoff, einen Rest $(CH_2CH_2O)_nR^1$ oder X, n für Zahlen von 1 bis 10 und X für Wasserstoff, ein Alkali- oder Erdalkalimetall oder $NR^3R^4R^5R^6$, mit $R^3$ bis $R^6$ unabhängig voneinander stehend für Wasserstoff oder einen C1 bis C4 - Kohlenwasserstoffrest, steht,
- sulfatierte Fettsäurealkylenglykolester der Formel (E1-II)

$$R^7CO(AlkO)_nSO_3M \qquad (E1\text{-}II)$$

in der $R^7CO$- für einen linearen oder verzweigten, aliphatischen, gesättigten und/oder ungesättigten Acylrest mit 6 bis 22 C-Atomen, Alk für $CH_2CH_2$, $CHCH_3CH_2$ und/oder $CH_2CHCH_3$, n für Zahlen von 0,5 bis 5 und M für ein Kation steht, wie sie in der DE-OS 197 36 906 beschrieben sind,
- Monoglyceridsulfate und Monoglyceridethersulfate der Formel (E1-III)

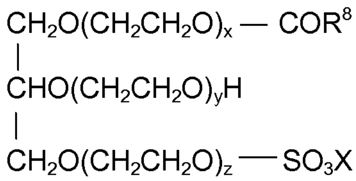
(E1-III)

in der $R^8CO$ für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der $R^8CO$ für einen linearen Acylrest mit 8 bis 18 Kohlenstoffatomen steht,
- Amidethercarbonsäuren,
- Kondensationsprodukte aus $C_8$ - $C_{30}$ - Fettalkoholen mit Proteinhydrolysaten und/oder Aminosäuren und deren Derivaten, welche dem Fachmann als Eiweissfettsäurekondensate bekannt sind, wie beispielsweise die Lamepon® - Typen, Gluadin® - Typen, Hostapon® KCG oder die Amisoft® - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Erfindungsgemäß einsetzbar sind weiterhin kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyl-trimethylammoniumchloride, Dialkyldimethylammoniumchloride und Trialkylmethylammonium-chloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationische Tenside sind die unter den INCI-Bezeichnungen Quaternium-27 und Quaternium-83 bekannten Imidazolium-Verbindungen.

Als zwitterionische Tenside werden solche oberflächenaktiven Verbindungen bezeichnet, die im Molekül mindestens eine quartäre Ammoniumgruppe und mindestens eine $-COO^{(-)}$ - oder $-SO_3^{(-)}$ -Gruppe tragen. Besonders ge-

eignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche oberflächenaktiven Verbindungen verstanden, die außer einer $C_8$ - $C_{24}$- Alkyl- oder -Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das $C_{12}$- $C_{18}$ - Acylsarcosin.

Die erfindungsgemäßen Mittel enthalten die Inhalts- bzw- Wirkstoffe in einem kosmetisch akzeptablen Träger. Bevorzugte kosmetisch akzeptable Träger sind wässrige, alkoholische oder wässrigalkoholische Medien mit vorzugsweise mindestens 10 Gew.-% Wasser, bezogen auf das gesamte Mittel. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Es ist erfindungsgemäß bevorzugt mindestens einen ($C_1$ bis $C_4$)-Monoalkylalkohol in den erfindungsgemäßen Mitteln insbesondere in einer Menge von 1 bis 50 Gew.-% insbesondere von 5 bis 30 Gew.-% einzusetzen. Dies ist wiederum insbesondere für die Konfektionierung als Pumpschaum oder Aerosolschaum bevorzugt.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gew.-% bezogen auf das gesamte Mittel.

Insbesondere der Zusatz von Glycerin und/oder Propylenglykol und/oder Polyethylenglykol und/oder Polypropylenglykol erhöht die Flexibilität des bei Anwendung des erfindungsgemäßen Mittels gebildeten Polymerfilms. Wird also ein flexibler Halt gewünscht, enthalten die erfindungsgemäßen Mittel vorzugsweise 0,01 bis 30 Gew.-% Glycerin und/oder Propylenglykol und/oder Polyethylenglykol und/oder Polypropylenglykol bezogen auf das gesamte Mittel.

Die Mittel weisen bevorzugt einen pH-Wert von 2 bis 11 auf. Besonders bevorzugt ist der pH-Bereich zwischen 2 und 8. Die Angaben zum pH-Wert beziehen sich dabei im Sinne dieser Schrift auf den pH-Wert bei 25°C, sofern nichts anderes vermerkt ist.

Die erfindungsgemäßen Mittel können weiterhin die Hilfs- und Zusatzstoffe enthalten, die üblicherweise herkömmlichen Stylingmitteln zugesetzt werden.

Als geeignete Hilfs- und Zusatzstoffe sind insbesondere zusätzliche Pflegestoffe zu nennen.

Als Pflegestoff kann beispielsweise ein Silikonöl und/oder ein Silikongum eingesetzt werden.

Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Silikonöle bewirken die unterschiedlichsten Effekte. So beeinflussen sie beispielsweise gleichzeitig die Trocken- und Nasskämmbarkeiten, den Griff des trockenen und nassen Haares sowie den Glanz. Unter dem Begriff Silikonöle versteht der Fachmann mehrere Strukturen Silicium-organischer Verbindungen. Zunächst werden hierunter die Dimethiconole verstanden. Als Beispiele für derartige Produkte werden die folgenden Handelsprodukte genannt: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401 DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS (beide Guardian Laboratories), Nonychosine E, Nonychosine V (beide Exsymol), SanSurf Petrolatum-25, Satin Finish (beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical

Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Dimethicone bilden die zweite Gruppe der Silikone, welche erfindungsgemäß enthalten sein können. Diese können sowohl linear als auch verzweigt als auch cyclisch oder cyclisch und verzweigt sein.

Dimethiconcopolyole (S3) bilden eine weitere Gruppe von Silikonen, die geeignet sind. Entsprechende Dimethiconcopolyole sind kommerziell erhältlich und werden beispielsweise von der Firma Dow Corning unter der Bezeichnung Dow Corning® 5330 Fluid vertrieben.

Selbstverständlich umfasst die erfindungsgemäße Lehre auch, dass die Dimethiconole, Dimethicone und/oder Dimethiconcopolymere bereits als Emulsion vorliegen können. Dabei kann die entsprechende Emulsion der Dimethiconole, Dimethicone und/oder Dimethiconcopolyole sowohl nach der Herstellung der entsprechenden Dimethiconole, Dimethicone und/oder Dimethiconcopolyole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconole, Dimethicone und/oder Dimethiconcopolyole auch direkt durch ein Emulsionspolymerisationsverfahren hergestellt werden. Auch derartige Verfahren sind dem Fachmann wohl bekannt.

Wenn die Dimethiconole, Dimethicone und/oder Dimethiconcopolyole als Emulsion verwendet werden, dann beträgt die Tröpfchengröße der emulgierten Teilchen erfindungsgemäß 0,01 bis 10000 $\mu$m, bevorzugt 0,01 bis 100 $\mu$m, besonders bevorzugt 0,01 bis 20 $\mu$m und ganz besonders bevorzugt 0,01 bis 10 $\mu$m. Die Teilchengröße wird dabei nach der Methode der Lichtstreuung bestimmt.

Werden verzweigte Dimethiconole, Dimethicone und/oder Dimethiconcopolyole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Erfindung ist daher unter verzweigten Dimethiconolen, Dimethiconen und/oder Dimethiconcopolyolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconole, Dimethicone und/oder Dimethiconcopolyole ganz besonders bevorzugt sein.

Besonders geeignete Silikone sind aminofunktionelle Silikone, insbesondere die Silikone, die unter der INCI-Bezeichnung Amodimethicone zusammengefasst sind. Daher ist es erfindungsgemäß bevorzugt, wenn die erfindungsgemäß Mittel zusätzlich mindestens ein aminofunktionelles Silikon enthalten. Darunter sind Silikone zu verstehen, welche mindestens eine, gegebenenfalls substituierte, Aminogruppe aufweisen. Diese Silikone werden nach der INCI-Deklaration als Amodimethicone bezeichnet und sind beispielsweise in Form einer Emulsion als Handelsprodukt Dow Corning® 939 oder als Handelsprodukt Dow Corning® 949 im Gemisch mit einem kationischen und eine nichtionischen Tensid erhältlich.

Vorzugsweise werden solche aminofunktionellen Silikone eingesetzt, die eine Aminzahl oberhalb von 0,25 meq/g, vorzugsweise oberhalb von 0,3 meq/g und insbesondere bevorzugt oberhalb von 0,4 meq/g aufweisen. Die Aminzahl steht dabei für die Milli-Äquivalente Amin pro Gramm des aminofunktionellen Silikons. Sie kann durch Titration ermittelt und auch in der Einheit mg KOH/g angegeben werden.

Die Mittel enthalten die Silikone bevorzugt in Mengen von 0,01 Gew.-% bis 15 Gew.-%, besonders bevorzugt von 0,05 bis 2 Gew.-%, bezogen auf das gesamte Mittel.

Als Pflegestoff einer anderen Verbindungsklasse kann das Mittel beispielsweise mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex), Sericin (Pentapharm) und Kerasol® (Croda) vertrieben.

Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Als Pflegestoff kann das erfindungsgemäße Mittel weiterhin mindestens ein Vitamin, ein Provitamin, eine Vitaminvorstufe und/oder eines derer Derivate enthalten.

Dabei sind erfindungsgemäß solche Vitamine, Provitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin $A_1$) sowie das 3,4-Didehydroretinol (Vitamin $A_2$). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die Mittel enthalten die Vitamin A-Komponente bevorzugt in Mengen von 0,05-1 Gew.-%, bezogen auf die gesamte Anwendungszubereitung.

Zur Vitamin B-Gruppe oder zu dem Vitamin B-Komplex gehören u. a. Vitamin $B_1$ (Thiamin), Vitamin $B_2$ (Riboflavin), Vitamin $B_3$ (Nicotinsäure und/oder Nicotinsäureamid (Niacinamid)), Vitamin $B_5$ (Pantothensäure, Panthenol und Pantolacton), Vitamin $B_6$ (Pyridoxin sowie Pyridoxamin und Pyridoxal), Vitamin C (Ascorbinsäure), Vitamin E (Tocopherole, insbesondere α-Tocopherol), Vitamin F (Linolsäure und/oder Linolensäure), Vitamin H.

Bevorzugt enthalten die erfindungsgemäßen Mittel Vitamine, Provitamine und Vitaminvorstufen aus den Gruppen A, B, C, E und H. Panthenol, Pantolacton, Pyridoxin und seine Derivate sowie Nicotinsäureamid und Biotin sind besonders bevorzugt.
Ganz besonders bevorzugt wird als Pflegestoff D-Panthenol, gegebenenfalls in Kombination mit mindestens einem der oben genannten Silikonderivate eingesetzt.

Wie auch der Zusatz von Glycerin und/oder Propylenglykol erhöht der Zusatz von Panthenol die Flexibilität des bei Anwendung des erfindungsgemäßen Mittels gebildeten Polymerfilms. Wird also ein besonders flexibler Halt gewünscht, können die erfindungsgemäßen Mittel statt oder zusätzlich zu Glycerin und/oder Propylenglykol Panthenol enthalten. In einer bevorzugten Ausführungsform enthalten die erfindungsgemäßen Mittel Panthenol, vorzugsweise in einer Menge von 0,05 bis 10 Gew.-%, besonders bevorzugt 0,1 - 5 Gew.-%, jeweils bezogen auf das gesamte Mittel.

Als Pflegestoff können die erfindungsgemäßen Mittel weiterhin mindestens einen Pflanzenextrakt enthalten. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.
Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Weiterhin kann es bevorzugt sein, in den erfindungsgemäßen Mitteln Mischungen aus mehreren, insbesondere aus zwei, verschiedenen Pflanzenextrakten einzusetzen.

Auch Mono- bzw. Oligosaccharide können als Pflegestoff in den erfindungsgemäßen Mitteln eingesetzt werden. Es können sowohl Monosaccharide als auch Oligosaccharide, wie beispielsweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten.
Geeignete Pentosen und Hexosen sind beispielsweise Ribose, Arabinose, Xylose, Lyxose, Allose, Altrose, Glucose, Mannose, Gulose, Idose, Galactose, Talose, Fucose und Fructose. Arabinose, Glucose, Galactose und Fructose sind bevorzugt eingesetzte Kohlenhydrate; Ganz besonders bevorzugt eingesetzt wird Glucose, die sowohl in der D-(+)- oder L-(-)- Konfiguration oder als Racemat geeignet ist.
Weiterhin können auch Derivate dieser Pentosen und Hexosen, wie die entsprechenden On- und Uronsäuren (Zuckersäuren), Zuckeralkohole und Glykoside, erfindungsgemäß eingesetzt werden. Bevorzugte Zuckersäuren sind die Gluconsäure, die Glucuronsäure, die Zuckersäure, die Mannozuckersäure und die Schleimsäure. Bevorzugte Zuckeralkohole sind Sorbit, Mannit und Dulcit. Bevorzugte Glykoside sind die Methylglucoside.
Da die eingesetzten Mono- bzw. Oligosaccharide üblicherweise aus natürlichen Rohstoffen wie Stärke gewonnen werden, weisen sie in der Regel die diesen Rohstoffen entsprechenden Konfigurationen auf (z.B. D-Glucose, D-Fructose und D-Galactose).
Die Mono- bzw. Oligosaccharide sind in den erfindungsgemäßen Mitteln bevorzugt in einer Menge von 0,1 bis 8 Gew.-%, insbesondere bevorzugt 1 bis 5 Gew.-%, bezogen auf die gesamte Anwendungszubereitung, enthalten.

Das Mittel kann weiterhin mindestens ein Lipid als Pflegestoff enthalten.

Erfindungsgemäß geeignete Lipide sind Phospholipide, beispielsweise Sojalecithin, Ei-Lecithin und Kephaline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA®, Phospholipid PTC® sowie Phospholipid SV® vertrieben. Die erfindungsgemäßen Mittel enthalten die Lipide bevorzugt in Mengen von 0,01 bis 10 Gew.-%, insbesondere 0,1 bis 5 Gew.-%, bezogen auf die gesamte Anwendungszubereitung.

Weiterhin sind als Pflegestoff Ölkörper geeignet.

Zu den natürlichen und synthetischen kosmetischen Ölkörpern sind beispielsweise zu zählen:

- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyln-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol®OE) können bevorzugt sein.
- Esteröle. Unter Esterölen sind zu verstehen die Ester von $C_6$ - $C_{30}$ - Fettsäuren mit $C_2$ - $C_{30}$ - Fettalkoholen. Bevorzugt sind die Monoester der Fettsäuren mit Alkoholen mit 2 bis 24 C-Atomen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Trifettsäureester von gesättigten und/oder ungesättigten linearen und/oder verzweigten Fettsäuren mit Glycerin,
- Fettsäurepartialglyceride, worunter Monoglyceride, Diglyceride und deren technische Gemische zu verstehen sind. Bei der Verwendung technischer Produkte können herstellungsbedingt noch geringe Mengen Triglyceride enthalten sein. Die Partialglyceride folgen vorzugsweise der Formel (D4-I),

$$\begin{array}{l} CH_2O(CH_2CH_2O)_mR^1 \\ | \\ CHO(CH_2CH_2O)_nR^2 \\ | \\ CH_2O(CH_2CH_2O)_qR^3 \end{array} \qquad \text{(D4-I)}$$

in der $R^1$, $R^2$ und $R^3$ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht $R^1$ für einen Acylrest und $R^2$ und $R^3$ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäßen Mitteln beträgt üblicherweise 0,1 - 30 Gew.%, bezogen auf die gesamte Anwendungszubereitung, bevorzugt 0,1 - 20 Gew.-%, und insbesondere 0,1 - 15 Gew.-%.

Obwohl jeder der genannten Pflegestoffe für sich alleine bereits ein zufrieden stellendes Resultat ergibt, sind im Rahmen der vorliegenden Erfindung auch alle Ausführungsformen umfasst, in denen das Mittel mehrere Pflegestoffe auch aus verschiedenen Gruppen enthält.

Durch Zugabe eines UV-Filters können sowohl die Mittel selbst, als auch die behandelten Fasern vor schädlichen Einflüssen von UV-Strahlung geschützt werden. Vorzugsweise wird daher dem Mittel mindestens ein UV-Filter zugegeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders

bevorzugt.
Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.

In einer besonderen Ausführungsform enthält das erfindungsgemäße Mittel weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird.

Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 11, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol. Bevorzugt werden kationische direktziehende Farbstoffe eingesetzt. Besonders bevorzugt sind dabei

(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Die Farbstoffe, die auch unter den Bezeichnungen Basic Yellow 87, Basic Orange 31 und Basic Red 51 bekannt sind, sind ganz besonders bevorzugte kationische direktziehende Farbstoffe der Gruppe (c). Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.
Die erfindungsgemäßen Mittel gemäß dieser Ausführungsform enthalten die direktziehenden Farbstoffe bevorzugt in einer Menge von 0,001 bis 20 Gew.-%, bezogen auf das gesamte Mittel.

Es ist erfindungsgemäß bevorzugt, dass die erfindungsgemäßen Mittel frei von Oxidationsfarbstoffvorprodukten sind. Oxidationsfarbstoffvorprodukte werden eingeteilt in sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Die Formulierung der erfindungsgemäßen Mittel kann in allen für Stylingmittel üblichen Formen erfolgen, beispielsweise in Form von Lösungen, die als Haarwasser oder Pump- oder Aerosolspray auf das Haar aufgebracht werden können, in Form von Cremes, Emulsionen, Wachsen, Gelen oder auch tensidhaltigen schäumenden Lösungen oder anderen Zubereitungen, die für die Anwendung auf dem Haar geeignet sind.

Haarcremes und Haargele enthalten in der Regel Strukturanten und/oder verdickende Polymere, die dazu dienen, den Produkten die gewünschte Konsistenz zu verleihen. Strukturanten und/oder verdickende Polymere werden typischerweise in einer Menge von 0,1 bis 10 Gew.-%, bezogen auf das gesamte Produkt, eingesetzt. Mengen von 0,5 bis 5 Gew.-%, insbesondere 0,5 bis 3 Gew.-% sind bevorzugt.

Vorzugsweise werden die erfindungsgemäßen Mittel als Pumpspray, Aerosolspray, Pumpschaum oder Aerosolschaum konfektioniert.

Hierzu werden die erfindungsgemäßen Mittel in einer Abgabevorrichtung konfektioniert, die entweder ein zusätzlich mit einem Treibmittel befüllter Druckgasbehälter ("Aerosolbehälter") oder ein Nichtaerosolbehälter darstellt.

Die Druckgasbehälter, mit deren Hilfe ein Produkt durch den inneren Gasdruck des Behälters über ein Ventil verteilt wird, bezeichnet man definitionsgemäß als "Aerosolbehälter". Als "Nichtaerosolbehälter" wird im Umkehrschluß

zur Aerosoldefinition ein Behältnis unter Normaldruck definiert, mit dessen Hilfe ein Produkt mittels mechanischer Einwirkung durch ein Pumpsystem verteilt wird.

Insbesondere bevorzugt sind die erfindungsgemäßen Mittel als Aerosolhaarschaum oder Aerosolhaarspray konfektioniert. Das erfindungsgemäße Mittel enthält daher bevorzugt zusätzlich mindestens ein Treibmittel.

Erfindungsgemäß geeignete Treibmittel sind beispielsweise ausgewählt aus $N_2O$, Dimethylether, $CO_2$, Luft, Alkanen mit 3 bis 5 Kohlenstoffatomen, wie Propan, n-Butan, iso-Butan, n-Pentan und iso-Pentan, und deren Mischungen. Bevorzugt sind Dimethylether, Propan, n-Butan, iso-Butan und Mischungen daraus.

Gemäß einer bevorzugten Ausführungsform werden die genannten Alkane, Mischungen der genannten Alkane oder Mischungen der genannten Alkane mit Dimethylether als einziges Treibmittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe.

Über das Mengenverhältnis von Treibmittel zu den übrigen Bestandteilen der Zubereitungen lassen sich bei gegebener Sprühvorrichtung die Größen der Aerosoltröpfchen bzw. der Schaumblasen und die jeweilige Größenverteilung einstellen.

Die Menge an eingesetztem Treibmittel variiert in Abhängigkeit von der konkreten Zusammensetzung des Mittels, der verwendeten Verpackung und der gewünschten Produktart, etwa Haarspray oder Haarschaum. Bei Verwendung herkömmlicher Sprühvorrichtungen enthalten Aerosolschaumprodukte das Treibmittel bevorzugt in Mengen von 1 bis 35 Gew.-%, bezogen auf das gesamte Produkt. Mengen von 2 bis 30 Gew.-%, insbesondere von 3 bis 15 Gew.-% sind besonders bevorzugt. Aerosolsprays enthalten generell größere Mengen an Treibmittel. Bevorzugt wird das Treibmittel in diesem Fall in einer Menge von 30 bis 98 Gew.-%, bezogen auf das gesamte Produkt, eingesetzt. Mengen von 40 bis 95 Gew.-%, insbesondere von 50 bis 95 Gew.-% sind besonders bevorzugt.

Die Aerosolprodukte lassen sich in üblicher Art und Weise herstellen. In der Regel werden alle Bestandteile des jeweiligen Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.

Zur Verschäumung von gelförmigen Mitteln in einem Zweikammer-Aerosolbehälter eignet sich bevorzugt Isopentan als ein Treibmittel, welches in die erfindungsgemäßen Mittel eingearbeitet wird und in der ersten Kammer des Zweikammer-Aerosolbehälters konfektioniert ist. In der zweiten Kammer des Zweikammer-Aerosolbehälters wird mindestens ein weiteres, von Isopentan verschiedenes Treibmittel konfektioniert, welches in dem Zweikammer-Aerosolbehälter einen höheren Druck aufbaut als das Isopentan. Die Treibmittel der zweiten Kammer werden bevorzugt ausgewählt aus $N_2O$, Dimethylether, $CO_2$, Luft, Alkanen mit 3 oder 4 Kohlenstoffatomen (wie Propan, n-Butan, iso-Butan) sowie Mischungen daraus.

Eine bevorzugte Ausführungsform der erfindungsgemäßen Mittel sind Aerosolhaarschäume oder Aerosolhaarsprays, enthaltend das zuvor beschriebene erfindungsgemäße Mittel und mindestens ein Treibmittel.

Bevorzugte erfindungsgemäße Mittel und Treibmittel des Aerosolhaarschaums bzw. Aerosolhaarsprays, sowie die jeweiligen Mengen an Treibmittel entsprechen dem bereits oben Ausgeführten.

Ein zweiter Gegenstand der Erfindung ist die Verwendung der erfindungsgemäßen Mittel zur temporären Verformung von Haaren und/oder zur Haarpflege.

Die erfindungsgemäßen Mittel und Produkte, die diese Mittel enthalten, insbesondere Aerosolhaarschäume bzw. Aerosolhaarsprays, zeichnen sich insbesondere dadurch aus, dass sie behandeltem Haar einen sehr starken, dauerhaften Frisurenhalt verleihen, obgleich das Haar bleibt flexibel bleibt. Wird das Mittel als Haarschaum konfektioniert, bildet sich ein stabiler, feinporiger und cremiger Schaum, der sich gleichmäßig und ohne zu tropfen auf dem Haar verteilen lässt.

Ein dritter Gegenstand der Erfindung ist ein Verfahren zur Behandlung keratinhaltiger Fasern, insbesondere menschlicher Haare, worin unter Einsatz einer Abgabevorrichtung ein Mittel gemäß erstem Erfindungsgegenstand zu einem Schaum verschäumt wird und der resultierende Schaum auf die keratinhaltigen Fasern appliziert wird.

Dabei ist es erfindungsgemäß bevorzugt, dass die keratinhaltigen Fasern in Form gebracht werden und diese Form durch das Mittel des ersten Erfindungsgegenstandes fixiert wird.

Als erfindungsgemäß bevorzugt gelten die zuvor genannten Abgabevorrichtungen (*vide supra*).

Ein vierter Gegenstand der Erfindung ist ein Verfahren zur Behandlung keratinhaltiger Fasern, insbesondere menschlicher Haare, worin unter Einsatz einer Abgabevorrichtung ein Mittel gemäß erstem Erfindungsgegenstand als Spray auf die keratinhaltigen Fasern appliziert wird.

Dabei ist es erfindungsgemäß bevorzugt, dass die keratinhaltigen Fasern in Form gebracht werden und diese Form durch das Mittel des ersten Erfindungsgegenstandes fixiert wird.

Als erfindungsgemäß bevorzugt gelten die zuvor genannten Abgabevorrichtungen *(vide supra).*

Die nachfolgenden Beispiele sollen den Gegenstand der vorliegenden Erfindung erläutern ohne ihn in irgendeiner Weise zu beschränken.

**Beispiele**

Falls nicht anderweitig definiert wurden die Mengenangaben als Gewichtsprozent gemessen. Folgende Rezepturen der Tabellen 1 und 2 wurden hergestellt:

Tabelle 1:

| | A | B | C | D | E | F | G | H | I |
|---|---|---|---|---|---|---|---|---|---|
| HPS [1] | 4,0 | 5,0 | 4,5 | 3,0 | 6,0 | 3,5 | 5,5 | 6,5 | 5,0 |
| PVP/VA [2] | - | - | - | 5,0 | - | - | 4,0 | - | - |
| Polyvinylpyrrolidon | - | - | - | - | 0,5 | - | 1,0 | - | - |
| Polyquaternium-4 | 0,5 | 0,2 | - | 0,2 | - | - | 0,8 | - | - |
| Polyquaternium-55 | - | - | - | - | - | 1,0 | - | - | - |
| Polyquaternium-11 | - | 1,5 | - | - | 2,0 | - | - | - | - |
| Polyquaternium-69 | 2,0 | - | - | - | - | 0,3 | - | - | - |
| Polyquaternium-68 | - | - | 2,0 | - | - | - | - | - | - |
| Polyquaternium-16 | - | - | - | - | 1,0 | - | - | - | - |
| Polyquaternium-46 | - | - | - | - | - | - | - | - | 8,0 |
| Styleze CC-10 | - | - | - | - | - | - | - | 1,0 | - |
| Cetyltrimethylammoniumchlorid | 0,2 | 0,2 | 0,2 | 0,1 | 0,2 | 0,1 | 0,1 | 0,2 | 0,2 |
| Ethanol | 10,0 | - | - | 15,0 | - | 10,0 | - | - | 5,0 |
| Wasser | <------------------ ad 100 ------------------> | | | | | | | | |

Tabelle 2:

| | J | K | L | M | N | O | P | Q | R |
|---|---|---|---|---|---|---|---|---|---|
| HPS 2 [3] | 4,0 | 5,0 | 4,5 | 3,0 | 6,0 | 3,5 | 5,5 | 6,5 | 5,0 |
| PVP/VA [2] | - | - | - | 5,0 | - | - | 4,0 | - | - |
| Polyvinylpyrrolidon | - | - | - | - | 0,5 | - | 1,0 | - | - |
| Polyquaternium-4 | 0,5 | 0,2 | - | 0,2 | - | - | 0,8 | - | - |
| Polyquaternium-55 | - | - | - | - | - | 1,0 | - | - | - |
| Polyquaternium-11 | - | 1,5 | - | - | 2,0 | - | - | - | - |
| Polyquaternium-69 | 2,0 | - | - | - | - | 0,3 | - | - | - |
| Polyquaternium-68 | - | - | 2,0 | - | - | - | - | - | - |
| Polyquaternium-16 | - | - | - | - | 1,0 | - | - | - | - |
| Polyquaternium-46 | - | - | - | - | - | - | - | - 8,0 | 8,0 |
| Styleze CC-10 | - | - | - | - | - | - | - | 1,0 | - |
| Cetyltrimethylammoniumchlorid | 0,2 | 0,2 | 0,2 | 0,1 | 0,2 | 0,1 | 0,1 | 0,2 | 0,2 |
| Ethanol | 10,0 | - | - | 15,0 | - | 10,0 | - | - | 5,0 |

(fortgesetzt)

| | J | K | L | M | N | O | P | Q | R |
|---|---|---|---|---|---|---|---|---|---|
| Wasser | <-------------------------- ad 100 --------------------------> | | | | | | | | |
| [1] mittels Propylenoxid modifizierte Kartoffelstärke (Propylenoxidgehalt: 10,0 Gew.-%; Viskosität: 64000 mPas; Gewichtsmittelbereich: 700-900 kDa)<br>[2] Copolymer aus N-Vinylpyrrolidon und Vinylacetat<br>[3] mittels Propylenoxid modifizierte Kartoffelstärke (Propylenoxidgehalt: 5,0 Gew.-%; Viskosität: 128000 mPas; Gewichtsmittelbereich: 700-900 kDa) | | | | | | | | | |

Die Rezepturen A bis R wurden jeweils in ein Aerosolbehältnis gefüllt, das folgende technische Parameter erfüllt: Aluminium Vorratsbehältnis mit Ventil Produkt 522983 PV10697 der Firma Pecision (Deutsche Präzisions-Ventil GmbH).
Das Aerosolbehältnis wurde mit einem Treibgasgemisch aus Propan/Butan (47 Gew.-% Propan, 50 Gew.-% Butan, 3 Gew.-% Isobutan) befüllt, sodass sich ein Gewichtsverhältnis von Rezeptur zu Treibgas von 92 zu 8 ergab.
Alle Rezepturen bewirkten nach der Anwendung auf dem Haar eine Volumensteigerung sowie einen hervorragenden flexiblen Frisurenhalt. Das Haar erhielt eine sehr gute Pflege. Die Rezepturen wurden in Form eines voluminösen Aerosolschaums ausgebracht, der erst während der Anwendung am Haar signifikant bricht.

**Patentansprüche**

**1.** Mittel zur Behandlung keratinhaltiger Fasern, insbesondere menschlichem Haar, enthaltend in einem kosmetisch akzeptablen Träger

(a) mindestens eine nichtionische, mittels Propylenoxid modifizierte Stärke, wobei die nichtionische, mittels Propylenoxid modifizierte Stärke ein mittleres Molekulargewicht (Gewichtsmittel) von 700 bis 1000 kDa aufweist und wobei die nichtionische, mittels Propylenoxid modifizierte Stärke in einer Menge von 0,1 Gew.-% bis 10 Gew.-%, bezogen auf das Gewicht des Mittels, enthalten ist,
und
(b) mindestens ein filmbildendes kationisches und/oder festigendes kationisches Polymer.

**2.** Mittel nach Anspruch 1, **dadurch gekennzeichnet, dass** die nichtionische, mittels Propylenoxid modifizierte Stärke eine nichtionische, mittels Propylenoxid modifizierte Tapioka Stärke oder eine nichtionische, mittels Propylenoxid modifizierte Kartoffelstärke oder ein Gemisch beider vorgenannter Stärken ist.

**3.** Mittel nach einem der Ansprüche 1 oder 2, **dadurch gekennzeichnet, dass** die nichtionische, mittels Propylenoxid modifizierte Stärke in einer Menge von 0,2 Gew.-% bis 5,0 Gew.-%, ganz besonders bevorzugt von 1,0 bis 3,0 Gew.-%, jeweils bezogen auf das Gewicht des Mittels, enthalten ist.

**4.** Mittel nach einem der Ansprüche 1 bis 3, **dadurch gekennzeichnet, dass** die nichtionische, mittels Propylenoxid modifizierte Stärke in einer 43 Gew.-%-igen wässrigen Lösung eine Viskosität im Bereich von 150 bis 1500000 mPa·s. bevorzugt von 10000 bis 200000 mPa·s, besonders bevorzugt von 25000 bis 180000 mPa·s, (jeweils Brookfield Viskosimeter, Spindel #7 bei 20°C und 20 U/min) aufweist.

**5.** Mittel nach einem der Ansprüche 1 bis 4, **dadurch gekennzeichnet, dass** die nichtionische, mittels Propylenoxid modifizierte Stärke einen Propylenoxidgehalt von 1 bis 20 Gew.-%, bevorzugt einen Propylenoxidgehalt von 4 bis 12 Gew.-%, besonders bevorzugt einen Propylenoxidgehalt von 9,5 bis 10,5 Gew.-% oder von 4,0 bis 6,0 Gew.-%, - jeweils bezogen auf das Gewicht der modifizierten Stärke - aufweist.

**6.** Mittel nach einem der Ansprüche 1 bis 5, **dadurch gekennzeichnet, dass** als nichtionische, mittels Propylenoxid modifizierte Stärke mindestens eine unvernetzte, nichtionische, mittels Propylenoxid modifizierte Stärke enthalten ist.

**7.** Mittel nach einem der Ansprüche 1 bis 6, **dadurch gekennzeichnet, dass** die filmbildenden kationischen und/oder festigenden kationischen Polymere (b) in einer Menge von 0,1 Gew.-% bis 20,0 Gew.-%, besonders bevorzugt von

0,2 Gew.-% bis 10,0 Gew.-%, ganz besonders bevorzugt von 0,5 Gew.-% bis 5,0 Gew.-%, jeweils bezogen auf das Gewicht des erfindungsgemäßen Mittels, enthalten sind.

**8.** Mittel nach einem der Ansprüche 1 bis 7, **dadurch gekennzeichnet, dass** mindestens ein zusätzliches filmbildendes kationisches und/oder festigendes kationisches Polymer (b) enthalten ist, das mindestens ein Strukturelement der Formel (M1) enthält

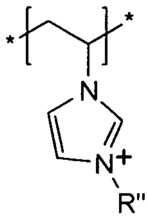
(M1)

worin

R'' für eine ($C_1$ bis $C_4$)-Alkylgruppe, insbesondere eine Methylgruppe, steht,
und zusätzlich mindestens ein weiteres kationisches und/oder nichtionisches Strukturelement aufweist.

**9.** Mittel nach einem der Ansprüche 1 bis 8, **dadurch gekennzeichnet, dass** zusätzliche filmbildende kationische und/oder festigende kationische Polymere (b) ausgewählt werden, aus mindestens einem kationischen, quaternisierten Cellulose-Derivat.

**10.** Mittel nach einem der Ansprüche 1 bis 9, **dadurch gekennzeichnet, dass** mindestens ein zusätzliches filmbildendes kationisches und/oder festigendes kationisches Polymer (b) enthalten ist, das mindestens eine Struktureinheit der Formel (M5) und mindestens eine Struktureinheit der Formel (V) und gegebenenfalls mindestens eine Struktureinheit der Formel (VI) umfasst

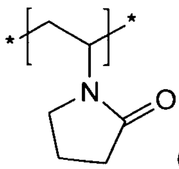
(M5)
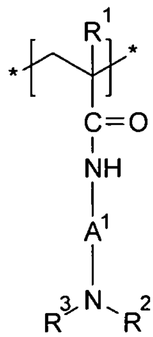
(V)
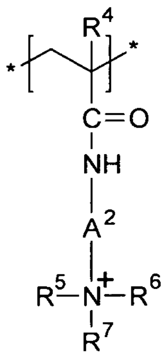
(VI)

worin

$R^1$ und $R^4$ stehen unabhängig voneinander für ein Wasserstoffatom oder eine Methylgruppe,
$A^1$ und $A^2$ stehen unabhängig voneinander für eine Gruppe Ethan-1,2-diyl, Propan-1,3-diyl oder Butan-1,4-diyl,
$R^2$, $R^3$, $R^5$ und $R^6$ stehen unabhängig voneinander für eine ($C_1$ bis $C_4$)-Alkylgruppe,
$R^7$ steht für eine ($C_8$ bis $C_{30}$)-Alkylgruppe.

**11.** Mittel nach einem der Ansprüche 1 bis 10, **dadurch gekennzeichnet, dass** zusätzlich mindestens ein filmbildendes, nichtionisches und/oder festigendes, nichtionisches Polymer (c) enthalten ist.

**12.** Mittel nach Anspruch 11, **dadurch gekennzeichnet, dass** als filmbildendes nichtionisches und/oder festigendes nichtionisches Polymer (c) mindestens ein Polymer ausgewählt aus der Gruppe, die gebildet wird aus

- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,

enthalten ist.

**13.** Mittel nach einem der Ansprüche 1 bis 12, **dadurch gekennzeichnet, dass** es in Form eines Aerosolschaums oder Aerosolsprays vorliegt.

**Claims**

**1.** An agent for treating keratin-containing fibers, in particular human hair, containing, in a cosmetically acceptable carrier,

(a) at least one non-ionic starch modified by means of propylene oxide, wherein the non-ionic starch modified by means of propylene oxide has an average molecular weight (weight average) of from 700 to 1000 kDa and wherein the non-ionic starch modified by means of propylene oxide is contained in an amount of from 0.1 wt.% to 10 wt.%, based on the weight of the agent,
and
(b) at least one film-forming cationic and/or strengthening cationic polymer.

**2.** The agent according to claim 1, **characterized in that** the non-ionic starch modified by means of propylene oxide is a non-ionic tapioca starch modified by means of propylene oxide or a non-ionic potato starch modified by means of propylene oxide or a mixture of the two aforementioned starches.

**3.** The agent according to claim 1 or 2, **characterized in that** the non-ionic starch modified by means of propylene oxide is contained in an amount of from 0.2 wt.% to 5.0 wt.%, most preferably from 1.0 wt.% to 3.0 wt.%, based on the weight of the agent in each case.

**4.** The agent according to one of claims 1 to 3, **characterized in that** the non-ionic starch modified by means of propylene oxide in a 43 wt.% aqueous solution has a viscosity in the range of from 150 to 1500000 mPa·s, preferably of from 10000 to 200000 mPa·s, particularly preferably of from 25000 to 180000 mPa·s (Brookfield viscometer, spindle #7 at 20 °C and 20 rpm in each case).

**5.** The agent according to one of claims 1 to 4, **characterized in that** the non-ionic starch modified by means of propylene oxide has a propylene oxide content of from 1 wt.% to 20 wt.%, preferably a propylene oxide content of from 4 to 12 wt.%, particularly preferably a propylene oxide content of from 9.5 wt.% to 10.5 wt.% or from 4.0 to 6.0 wt.%, based on the weight of the modified starch in each case.

**6.** The agent according to one of claims 1 to 5, **characterized in that** at least one uncrosslinked, non-ionic starch modified by means of propylene oxide is contained as the non-ionic starch modified by means of propylene oxide.

**7.** The agent according to one of claims 1 to 6, **characterized in that** the film-forming cationic and/or strengthening cationic polymers (b) are contained in an amount of from 0.1 wt.% to 20.0 wt.%, particularly preferably from 0.2 wt.% to 10.0 wt.%, most preferably from 0.5 wt.% to 5.0 wt.%, based on the weight of the agent according to the invention in each case.

**8.** The agent according to one of claims 1 to 7, **characterized in that** at least one additional film-forming cationic and/or strengthening cationic polymer (b) is contained, which polymer contains at least one structural element of the formula (M1)

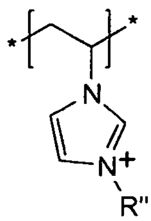

(M1)

where

R" denotes a ($C_1$ to $C_4$) alkyl group, particularly a methyl group,
and additionally comprises at least one further cationic and/or non-ionic structural element.

**9.** The agent according to one of claims 1 to 8, **characterized in that** additional film-forming cationic and/or strengthening cationic polymers (b) are selected from at least one cationic, quaternized cellulose derivative.

**10.** The agent according to one of claims 1 to 9, **characterized in that** at least one additional film-forming cationic and/or strengthening cationic polymer (b) is contained and comprises at least one structural unit of formula (M5) and at least one structural unit of formula (V) and optionally at least one structural unit of formula (VI),

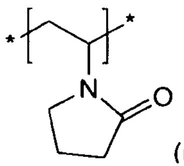
(M5)
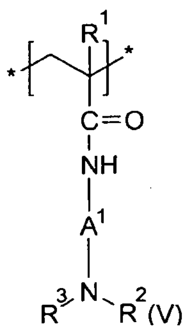
(V)
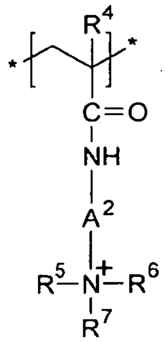
(VI)

where

$R^1$ and $R^4$ denote, independently of one another, a hydrogen atom or a methyl group,
$A^1$ and $A^2$ denote, independently of one another, an ethane-1,2-diyl, a propane-1,3-diyl or a butane-1,4-diyl group,
$R^2$, $R^3$, $R^5$ and $R^6$ denote, independently of one another, a ($C_1$ to $C_4$) alkyl group,
$R^7$ denotes a ($C_8$-$C_{30}$) alkyl group.

**11.** The agent according to one of claims 1 to 10, **characterized in that** at least one film-forming non-ionic and/or strengthening non-ionic polymer (c) is additionally contained.

**12.** The agent according to claim 11, **characterized in that**, as the film-forming non-ionic and/or strengthening non-ionic polymer (c), at least one polymer selected from the group formed by:

- polyvinylpyrrolidone,
- copolymers of N-vinylpyrrolidone and vinyl esters of carboxylic acids having 2 to 18 carbon atoms, particularly of N-vinylpyrrolidone and vinyl acetate

is contained.

**13.** The agent according to one of claims 1 to 12, **characterized in that** said agent is in the form of an aerosol mousse or an aerosol spray.

## Revendications

**1.** Agent de traitement de fibres kératinique, notamment du cheveu humain, comprenant dans un support cosmétiquement acceptable

(a) au moins un amidon non-ionique modifié à l'aide d'oxyde de propylène, celui-ci possédant une masse moléculaire moyenne (moyenne en poids) de 700 à 1 000 kDa et étant présent dans une quantité de 0,1 % en poids à 10 % en poids, par rapport au poids de l'agent,
et

(b) au moins un polymère cationique filmogène et/ou cationique fixant.

2. Agent selon la revendication 1, **caractérisé en ce que** l'amidon non-ionique modifié à l'aide d'oxyde de propylène est un amidon de tapioca non-ionique modifié à l'aide d'oxyde de propylène ou un amidon de pomme de terre non-ionique modifié à l'aide d'oxyde de propylène ou un mélange des deux amidons susmentionnés.

3. Agent selon une des revendications 1 ou 2, **caractérisé en ce que** l'amidon non-ionique modifié à l'aide d'oxyde de propylène est présent dans une quantité de 0,2 % en poids à 5,0 % en poids, de manière tout particulièrement préférée de 1,0 % en poids à 3,0 % en poids, par rapport au poids de l'agent respectivement.

4. Agent selon l'une quelconque des revendications 1 à 3, **caractérisé en ce que** l'amidon non-ionique modifié à l'aide d'oxyde de propylène possède, dans une solution aqueuse à 43 %, une viscosité allant de 150 à 1 500 000 mPa·s, de préférence de 10 000 à 200 000 mPa·s, de manière davantage préférée de 25 000 à 180 000 mPa·s (respectivement viscosimètre de Brookfield, fuseau #7 à une température de 20 °C et 20 U/min).

5. Agent selon l'une quelconque des revendications 1 à 4, **caractérisé en ce que** l'amidon non-ionique modifié à l'aide d'un oxyde de propylène présente une teneur en oxyde de propylène allant de 1 à 20 % en poids, de préférence une teneur en oxyde de propylène allant de 4 à 12 % en poids, de manière davantage préférée une teneur en oxyde de propylène allant de 9,5 à 10,5 % en poids ou de 4,0 à 6,0 % en poids - par rapport au poids de l'amidon modifié respectivement.

6. Agent selon l'une quelconque des revendications 1 à 5, **caractérisé en ce qu'**au moins un amidon non-ionique, non-réticulé, modifié à l'aide d'oxyde de propylène est présent comme amidon non-ionique modifié à l'aide d'oxyde de propylène.

7. Agent selon l'une quelconque des revendications 1 à 6, **caractérisé en ce que** les polymères (b) cationiques filmogènes et/ou cationiques fixants sont présents dans une quantité de 0,1 % en poids à 20,0 % en poids, de préférence de 0,2 % en poids à 10,0 % en poids, de manière davantage préférée de 0,5 % en poids à 5,0 % en poids, par rapport au poids de l'agent selon l'invention respectivement.

8. Agent selon l'une quelconque des revendications 1 à 7, **caractérisé en ce qu'**au moins un polymère (b) cationique filmogène et/ou cationique fixant supplémentaire est présent, qui comprend au moins un élément structurel de formule (M1)

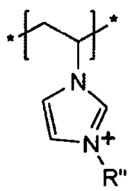

(M1)

où

R" représente un groupe alkyle (en $C_1$ à $C_4$), notamment un groupe méthyle,
et présente en plus au moins un autre élément structurel cationique et/ou non-ionique.

9. Agent selon l'une quelconque des revendications 1 à 8, **caractérisé en ce que** des polymères (b) cationiques filmogènes et/ou cationiques fixants sont sélectionnés parmi au moins un dérivé de cellulose quaternaire cationique.

10. Agent selon l'une quelconque des revendications 1 à 9, **caractérisé en ce qu'**au moins un polymère (b) cationique filmogène et/ou cationique fixant supplémentaire est présent, qui comprend au moins un motif structurel de formule (M5) et au moins un motif structurel de formule (V) et le cas échéant au moins un motif structurel de formule (VI)

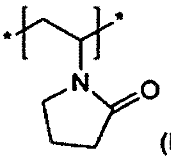 (M5)

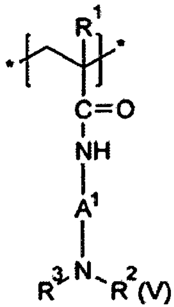 (V)

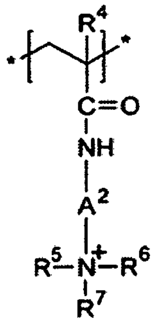 (VI)

où

$R^1$ et $R^4$ représentent indépendamment l'un de l'autre un atome d'hydrogène ou un groupe méthyle,
$A^1$ et $A^2$ représentent indépendamment l'un de l'autre un groupe d'éthane-1,2-diyl, de propane-1,3-diyl ou de butane-1,4-diyl,
$R^2$, $R^3$, $R^5$ et $R^6$ représentent indépendamment l'un de l'autre un groupe alkyle (en $C_1$ à $C_4$), $R^7$ représente un groupe alkyle (en $C_8$ à $C_{30}$).

**11.** Agent selon l'une quelconque des revendications 1 à 10, **caractérisé en ce qu'**en plus au moins un polymère (c) non-ionique filmogène et/ou non-ionique fixant est présent.

**12.** Agent selon la revendication 11, **caractérisé en ce qu'**au moins un polymère sélectionné parmi le groupe constitué de

- polyvinylpyrrolidone,
- copolymères de n-vinylpyrrolidone et d'esters de vinyle d'acides carboxyliques comportant 2 à 18 atomes de carbone, notamment de n-vinylpyrrolidone et d'acétate de vinyle,

est présent comme polymère (c) non-ionique filmogène et/ou non-ionique fixant.

**13.** Agent selon l'une quelconque des revendications 1 à 12, **caractérisé en ce qu'**il se présente sous la forme d'une mousse aérosol ou d'un spray aérosol.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE S19738866 O
- DE S19736906 O
- DE S19756454 O
- EP 998908 A2